# FASCICULE DE BREVET EUROPEEN

(11) **EP 1 519 952 B1**
(45) Date de publication et mention de la délivrance du brevet: **03.03.2010**
(21) Numéro de dépôt: 03763948.1
(22) Date de dépôt: 09.07.2003
(51) Int. Cl.: C07K 14/47, C12N 9/64, C12N 15/52, C07K 19/00, C07K 16/40, C07H 21/00, A61K 38/48, A61K 31/711, A61P 17/00, A61P 31/00, A61K 48/00, A61K 31/708

(54) **UTILISATION DE PROTEASES ASPARTIQUES DANS LE DOMAINE COSMETIQUE ET THERAPEUTIQUE**
VERWENDUNG VON ASPARTIC PROTEASEN IN DEN BEREICHEN KOSMETIK UND BEHANDLUNG
USE OF ASPARTIC PROTEASES IN COSMETICS AND THERAPEUTICS

(30) Priorité: 09.07.2002 FR 0208613
(43) Date de publication de la demande: 06.04.2005
(73) Titulaire: L'Oréal, 75008 Paris (FR)
(72) Inventeur: BERNARD, Dominique, F-75015 PARIS (FR); MEHUL, Bruno, F-06620 GOURDON (FR)
(74) Mandataire: Le Coupanec, Pascale A.M.P.
(86) Numéro de dépôt international: PCT/FR2003/002151
(87) Numéro de publication internationale: WO 2004/007548

(56) Documents cités:
- WO-A-02/22101
- DE-A- 19 950 020
- FR-A- 2 761 363
- US-A- 5 545 402
- US-B1- 6 274 364
- SUGANO S ET AL: "HOMO SAPIENS CDNA FLJ25084 FIS, CLONE CBL08511" EMBL, XP002235474
- ISOGAI T ET AL: "HOMO SAPIENS CDNA FLJ31432 FIS, CLONE NT2NE2000550" EMBL, XP002235475
- "1998 Biochemicals Catalog, PROTEASES FOR CLEAVAGE AND SEQUENCING" BOEHRINGER MANNHEIM BIOCHEMICALS CATALOG, XX, XX, 1998, pages 404-410, XP002235473
- RAWLINGS N D ET AL: "FAMILIES OF ASPARTIC PEPTIDASES, AND THOSE OF UNKNOWN CATALYTIC MECHANISM" METHODS IN ENZYMOLOGY, ACADEMIC PRESS INC, SAN DIEGO, CA, US, vol. 248, 1995, pages 105-120, XP008003749 ISSN: 0076-6879

## Description

La présente invention a pour objet principal l'utilisation, dans les domaines cosmétique et thérapeutique, des formes tronquées ou dérivées d'une protéase à acide aspartique dite SASPase ou d'un mélange de polypeptides issu de sa protéolyse notamment en vue de traiter les troubles liés à un dysfonctionnement de la prolifération et/ou de la différenciation cellulaire.

L'invention a également pour objet des séquences d'acide désoxyribonucléique codant les formes dites activées de ladite protéase à acide aspartique SASPase, les séquences polypeptidiques correspondantes et les utilisations desdites séquences désoxyribonucléiques.

Les protéases sont des enzymes hydrolytiques capables de couper des liaisons peptidiques. Un certain nombre d'entre elles sont aujourd'hui connues comme jouant un rôle essentiel au niveau de l'équilibre et de la physiologie de l'épiderme.

L'épiderme est conventionnellement divisé en une couche basale de kératinocytes constituant la couche germinative de l'épiderme, une couche dite épineuse constituée de plusieurs couches de cellules polyédriques disposées sur les couches germinatives, une à trois couches dites granuleuses constituées de cellules aplaties contenant des inclusions cytoplasmiques distinctes, les grains de kératohyaline et enfin, un ensemble de couches supérieures appelées couches cornées (ou *stratum corneum*), constituée de kératinocytes au stade terminal de leur différenciation appelés cornéocytes.

Les coméocytes sont des cellules anucléées principalement constituées d'une matière fibreuse contenant des cytokératines, entourée d'une enveloppe cornée. Il y a en permanence production de nouveaux kératinocytes pour compenser la perte en continu de cellules épidermiques au niveau de la couche cornée selon un mécanisme dénommé desquamation. Un déséquilibre entre la production des cellules au niveau de la couche basale et le taux de desquamation peut notamment conduire à des formations d'écailles à la surface de la peau.

En l'occurrence, de nombreuses pathologies cutanées se caractérisent par la production d'une couche cornée épaissie et par une desquamation anormale, c'est-à-dire par une hyperkératose. A titre d'exemple, on peut citer :
- la xérose (ou sécheresse cutanée),
- les ichthyoses,
- le psoriasis,
- certaines lésions tumorales bénignes ou malignes, et
- les hyperkératoses réactionnelles.

A l'inverse, certaines manifestations pathologiques entraînent un amincissement de l'épiderme et plus particulièrement de la couche cornée. Ce type de manifestations se traduit alors par une fragilité excessive du revêtement cutané. A titre représentatif de ces troubles, on peut notamment citer les réactions d'origine immunitaire généralement induites par mise en présence ou contact avec un ou plusieurs agents exogènes.

En conséquence, la connaissance des polypeptides impliqués dans la cohésion inter coméocytaire est une des voies qui peut permettre l'élaboration de produits destinés à lutter contre les effets d'un excès ou d'un défaut en polypeptide(s) de ce type, en particulier à la surface de la peau.

L'un des objets de l'invention est précisément de proposer l'utilisation dans un but cosmétique et/ou thérapeutique d'un polypeptide impliqué dans la régulation du phénomène de différenciation/prolifération épidermique.

Plus précisément, les inventeurs ont mis en évidence dans des kératinocytes humains, isolé et purifié un polypeptide possédant dans sa séquence peptidique (SEQ ID NO 5), la séquence FLVDSGAQVSVV (SEQ ID NO : 1) correspondant à une signature PROSITE PS00141 des sites actifs des protéases de la famille des protéases dites à acide aspartique.

Ce polypeptide encore dénommé ci-après protéine SASPase, est par ailleurs caractérisé par la présence dans sa séquence peptidique des séquences suivantes :
- AQFLVANASAEEAIIGTDVLQ (SEQ ID NO : 2) et
- ILGVWDTAV (SEQ ID NO: 3).

De manière inattendue les inventeurs ont mis en évidence que la protéine représentée par la séquence SEQ ID NO : 5, encore appelée protéine SASPase, possédait une activité protéolytique significative et ont notamment constaté cette activité vis-à-vis de la caséine et de l'insuline comme le montre les exemples ci-après.

Ils ont par ailleurs observé que cette protéine SASPase était autocatalytique et générait à un pH compris entre 3 et 7, et de préférence supérieur ou égal à 4,5 une forme tronquée dite « SASPase activée », correspondant à la séquence SEQ ID NO : 6 capable à son tour de se dimériser.

Un aspect de l'invention concerne donc un polypeptide isolé et purifié, appartenant à la famille des protéases à acide aspartique caractérisé en ce qu'il possède une séquence peptidique représentée par la séquence SEQ ID Na : 6, SEQ ID NO : 16, SEQ ID NO : 25 ou SEQ ID NO : 27.

La SEQ ID NO : 6 correspond à une forme activée de la SEQ ID NO : 5.

La SEQ ID NO : 16 correspond à la séquence SEQ ID NO : 6 délétée de ses deux premiers acides aminés.

La séquence SEQ ID NO: 25 est une autre forme activée de la SEQ ID NO : 5. Elle est obtenue à partir d'une forme tronquée de la SASPase (SEQ ID NO : 36) délétée du site codant pour la séquence FANS (SEQ ID NO : 29). Elle est plus particulièrement générée à pH 5.00 dans du tampon acétate.

La séquence SEQ ID NO : 27 correspond à la séquence SEQ ID NO : 25 délétée d'une partie de son fragment C-terminal.

D'une façon générale, l'invention s'étend à toutes les formes homologues des différents polypeptides ou séquences peptidiques cités. Classiquement, on entend par homologue d'un polypeptide ou d'une séquence peptidique, tout polypeptide ou toute séquence peptidique ayant une homologie de séquence d'au moins 85 %, notamment d'au moins 90 % et en particulier d'au moins 95 % et ayant le cas échéant le même type d'activité biologique que ledit polypeptide ou que ladite séquence peptidique.

Ces formes homologues englobent les variants définis ci-après.

L'invention s'étend en particulier aux formes homologues des polypeptides cités précédemment, c'est-à-dire manifestant la même activité biologique et possédant au moins 85 %, notamment au moins 90 % et en particulier au moins 95 % d'homologie de séquence avec la séquence SEQ ID NO : 6, SEQ ID NO : 16, SEQ ID NO : 25 ou SEQ ID NO : 27.

L'invention s'étend également aux protéines possédant à la fois le motif "aspartyl protéase rétroviral type" défini sous la référence de motif PROSITE : PS50175 ainsi qu'au moins un domaine transmembranaire tel que prédit par les algorithmes reconnus pour une telle détection parmi lesquels on peux citer: PRED-TMR2, TMHMM, TMpred et SOSUI.

Les modifications, dites encore mutations ou variations selon l'invention peuvent dériver, soit de la délétion d'un ou plusieurs acides aminés de la séquence SEQ ID NO : 6, SEQ ID NO: 16, SEQ ID NO: 25 ou SEQ ID NO: 27, ou de l'addition d'un ou plusieurs acides aminés à la séquence SEQ ID NO: 6, SEQ ID NO: 16, SEQ ID NO: 25
ou SEQ ID NO: 27, soit encore de la substitution d'un ou plusieurs acides aminés à la séquence SEQ ID NO: 6, SEQ ID NO: 16, SEQ ID NO: 25 ou SEQ ID NO: 27. Les séquences correspondantes sont encore désignées sous le terme de variants dans le cadre de la présente invention.

A titre illustratif des variants de délétion on peut plus particulièrement citer les séquences peptidiques suivantes:

La séquence SEQ ID NO: 4 correspondant à la SEQ ID NO: 5 tronquée de son fragment N-terminal Δ*1- 84*.

La séquence SEQ ID NO: 7 correspondant à la séquence de la partie N-terminale de la protéine SASPase (SEQ ID NO : 5). L'interaction de ce fragment avec un ligand biologique pourrait être une étape importante pour l'activation de la protéine SASPase et notamment la génération de sa forme activée (SEQ ID NO : 6).

La séquence SEQ ID NO : 8 correspondant à la séquence de la partie dite transmembranaire de la protéine SASPase (SEQ ID NO : 5).

La séquence SEQ ID NO : 9 correspondant à la séquence d'un peptide issu de la partie C-terminale de la protéine SASPase (SEQ ID NO : 5).

Les séquences SEQ ID NO : 29 et SEQ ID NO 30 correspondent à deux sites d'activation.

Sont également couverts sous le terme de variant, les protéines de fusion de la protéine SASPase (SEQ ID NO: 5), de ses formes activées SEQ ID NO: 6, SEQ ID NO : 16, SEQ ID NO : 25 ou SEQ ID NO : 27 avec un autre polypeptide, un agent de ciblage hydrophile ou hydrophobe ou un précurseur de bioconversion susceptible notamment de contrôler l'activation de ladite protéine.

Il est connu que les polypeptides peuvent subir des modifications post-traductionnelles comme la formation de liaisons disulfures, les clivages protéolytiques spécifiques, l'addition de glucides (glycosylation), la phosphorylation en particulier au niveau des sérines et/ou des thréonines et/ou des tyrosines, et/ou l'association à des lipides.

Le polypeptide de l'invention peut avoir subi une ou plusieurs modifications post-traductionnelles. En particulier, les polypeptides selon l'invention peuvent être N-glycosylés, phosphorylés, myristoylés, amidés et/ou citrullinés.

Ainsi, l'invention concerne également les polypeptides revendiqués ayant subi ou non des modifications post-traductionnelles.

L'invention s'étend également aux formes multimères et de préférence à la forme dimère de la séquence peptidique SEQ ID NO : 6, SEQ ID NO : 16, SEQ ID NO : 25 ou SEQ ID NO : 27. La forme dimère de la séquence peptidique SEQ ID NO : 6 est en particulier caractérisée en exemple V ci-après. Elle peut notamment être obtenue par association moléculaire de la forme monomérique ou par expression de son ADNc codant pour le dimère actif, incorporant à titre d'agent de liaison entre les deux entités monomériques, un motif composé de 2 à 6 acides aminés.

Les polypeptides revendiqués peuvent être d'origine naturelle ou synthétique. Par synthétique, on entend ici tout polypeptide obtenu chimiquement ou par production dans un organisme après introduction dans cet organisme des éléments nécessaires à cette production.

Ils peuvent être issus de toute origine possible à savoir soit animale, en particulier de mammifères et encore plus particulièrement humaine, soit végétale, soit de micro-organismes (par exemple virus, phages, bactéries, levures entre autres) ou encore de champignons, ou issu d'une surexpression dans un système eucaryote, par exemple une cellule de mammifère, sans préjuger du fait qu'ils soient présents de manière naturelle ou non dans ledit organisme d'origine.

En particulier, les polypeptides conformes à l'invention sont d'origine naturelle, purifiés à partir de tissus de mammifères, plus particulièrement à partir de peau de mammifères.

En particulier, ils sont purifiés à partir de peau humaine et encore plus particulièrement à partir d'épiderme humain.

On sait que dans un polypeptide, un ou plusieurs résidus d'acide aminé peuvent être remplacés par des résidus d'acide aminé ayant un indice hydropathique similaire sans pour autant changer les propriétés biologiques du polypeptide.

L'indice hydropathique est un indice attribué aux acides aminés en fonction de leur hydrophobicité et de leur charge (Kyte et al. (1982), J. Mol. Biol., 157 : 105).

Ainsi l'invention a également pour objet un polypeptide tel que décrit ci-dessus dans lequel un résidu d'acide aminé au moins a été remplacé par un résidu d'acide aminé ayant un indice hydropathique similaire.

On peut également classer les polypeptides en fonction de leur point isoélectrique.

Le point isoélectrique théorique d'un polypeptide peut être déduit de son enchaînement en acides aminés. Les polypeptides de l'invention sont théoriquement des polypeptides acides.

Ainsi les polypeptides de séquence SEQ ID NO: 5, SEQ ID NO : 6 ou SEQ ID NO: 16 possèdent un point isoélectrique compris entre 3 et 9, plus particulièrement entre 4 et 6 et notamment d'environ 5,8.

On sait en outre que la séquence primaire en acides aminés ainsi que les diverses modifications post-traductionnelles subies par un polypeptide font que ledit polypeptide peut être caractérisé par sa masse moléculaire apparente exprimée en kilodaltons.

On entend par masse moléculaire apparente, la masse moléculaire obtenue pour le polypeptide par comparaison de la mobilité électrophorétique de celui-ci avec celles de protéines standards de poids moléculaires connus sur gel de polyacrylamide/sodium dodécylsulfate, ou encore par comparaison du volume d'élution du polypeptide avec celui de protéines standard de poids moléculaires connus en chromatographie d'exclusion (selon les techniques décrites dans « Protein Purification », J-C. Janson et L. Ryden, VCH Publisher Inc. N.Y., 1989). (La méthode retenue dans le cadre de l'invention est celle reposant sur la mobilité électrophorétique).

La connaissance de l'enchaînement en acides aminés du polypeptide de l'invention permet d'en déterminer le poids moléculaire théorique.

L'invention concerne donc un polypeptide de séquence SEQ ID NO : 6 ayant une masse moléculaire apparente comprise entre 5 et 30 kilodaltons (kD), notamment entre 9 et 15 kD, et plus particulièrement entre 11 et 14 kD. En particulier, ce polypeptide de l'invention a une masse moléculaire apparente de l'ordre de 12kD.

Comme il ressort des exemples présentés ci-après, les inventeurs ont caractérisé l'expression du polypeptide dont la séquence est représentée par la séquence SEQ ID NO : 5 dans un grand nombre de tissus biologiques humains tel que le foie foetal, le placenta, le muscle, le poumon, l'intestin grêle et surtout au niveau du cerveau et du coeur et plus particulièrement au niveau de l'épiderme où l'expression est particulièrement élevée.

Il a par ailleurs été constaté que la SASPase de séquence SEQ ID NO : 5 dégradait la coméodesmosine, qui est un marqueur de la desquamation.

Enfin, la présence, dans la séquence polypeptidique de la SASPase (SEQ ID NO : 5) d'un site autocatalytique correspondant à un site décrit pour la protéase de type matrilysine capable d'activer les MMP de type 1, 2, et 9, témoigne d'une activité potentielle de la SASPase, ainsi que de ses formes activées (SEQ ID NO: 6, SEQ ID NO : 16, SEQ ID NO : 25 ou SEQ ID NO : 27) ou de ses fragments, sur des substrats endogènes et donc d'applications potentielles en cicatrisation, ré-épithialisation, vieillissement, angiogénèse et cancérisation (processus d'invasion) pour ladite protéine et ses différentes formes activées ou fragments.

L'ensemble de ces informations, valide donc l'implication du polypeptide conforme à l'invention dans le processus de prolifération et/ou différenciation cellulaire et identifie celui-ci en tant que nouvelle cible dermato/cosmétologique et thérapeutique.

En conséquence, un second aspect de l'invention concerne une composition cosmétique ou pharmaceutique comprenant, dans un milieu physiologiquement acceptable, au moins un polypeptide naturel ou synthétique purifié dont la séquence peptidique est représentée par une séquence choisie parmi SEQ ID NO: 6, SEQ ID NO : 16, SEQ ID NO : 25 et SEQ ID NO : 27 et leurs homologues possédant au moins 85 % d'homologie de séquence avec lesdites séquences et la même activité biologique.

La présente invention concerne également une composition cosmétique ou pharmaceutique comprenant au moins un polypeptide naturel ou synthétique purifié dont la séquence peptidique telle que définie précédemment est présente sous une forme multimère et de préférence dimère.

Au sens de l'invention, et sans indication contraire, on entend couvrir sous le terme polypeptide dans les compositions revendiquées les polypeptides naturels ou synthétiques, qu'il soit obtenu par protéolyse ou par synthèse, les différentes formes post-traductionnelles de ceux-ci et notamment celles décrites précédemment ou encore tout polypeptide naturel ou synthétique dont la séquence est totalement ou partiellement constituée par les séquences précitées comme par exemple les variants décrits ci-dessus.

La présente invention concerne également une composition cosmétique ou pharmaceutique dans laquelle ledit polypeptide se présente sous la forme d'un polypeptide de séquence SEQ ID NO: 6, SEQ ID NO : 16, SEQ ID NO : 25 ou SEQ ID NO : 27 fusionné avec un autre polypeptide, un agent de ciblage hydrophile ou hydrophobe ou un précurseur de bioconversion.

La quantité de polypeptide contenue dans les compositions de l'invention est, bien entendu, fonction de l'effet recherché et peut donc varier dans une large mesure.

Pour donner un ordre de grandeur, la composition peut contenir un polypeptide conforme à l'invention en une quantité représentant de 0,00001 % à 50 % du poids total de la composition et préférentiellement en une quantité représentant de 0,001 % à 10 % du poids total de la composition et encore plus préférentiellement en une quantité représentant de 0,1 % à 1 % du poids total de la composition.

Comme décrit précédemment, un certain nombre de désordres sont associés à des troubles de différenciation et/ou prolifération cellulaire. Dans la mesure où les polypeptides conformes à l'invention sont impliqués au niveau de la régulation de ces deux phénomènes, ils constituent avantageusement des cibles potentielles pour traiter tout désordre résultant d'un dysfonctionnement de la prolifération ou de la différenciation cellulaires en particulier épidermiques. En conséquence, outre le fait que les polypeptides selon l'invention peuvent être utilisés directement à titre de matière active dans une composition cosmétique ou pharmaceutique, ils peuvent également eux-mêmes servir de cible dans un traitement cosmétique ou pharmaceutique ou être utilisés à titre d'outils de diagnostic.

Les inventeurs ont par ailleurs caractérisé la présence de sites de coupure dans la séquence peptidique SEQ ID NO: 5, en partie localisés dans sa partie N-terminale et également présents dans les formes actives SEQ ID NO : 6, et SEQ ID NO : 16 et en partie présents dans sa partie C-terminale et également présents dans les formes actives SEQ ID NO: 25 et SEQ ID NO : 27. Plus précisément, les sites de coupure déduits de l'autoactivation de la SASPase elle-même sont F/A, N/S, E/L, A/L, R/F, H/S, F/E, E/A. A l'évidence, ces sites de coupure peuvent être déterminants au niveau de l'activation de la protéine, soit pour la bloquer, soit au contraire pour activer son hydrolyse.

Les sites pertinents plus précisément localisés dans la région N-terminale sont F/A et N/S et figurent dans la séquence FANS dite SEQ ID NO: 29. Ils semblent en particulier impliqués dans la génération de la forme activée' représentée par la SEQ ID NO: 25.

En ce qui concerne la seconde région potentielle d'activation ou d'inactivation de la protéine SASPase SEQ ID NO: 5, elle correspond plus particulièrement à la séquence DLELIE dite SEQ ID NO: 30 et comprend notamment le site de coupure E/L.

A partir de ces sites de coupure, on peut envisager d'effectuer la synthèse de différents types de peptides soit modifiés, soit portant un fluorophore quenché et qui serviront de substrat ou d'inhibiteurs.

Le minimum de séquence utilisable est évidemment le dipeptide (acides aminés de part et d'autre du site de coupure) mais en général on utilise des peptides de 8 à 12 acides aminés où le site de coupure est en position centrale. Par exemple, on sait que la SASPase coupe l'insuline en position E/A. On peut donc imaginer de développer un substrat de type peptidique incorporant ce site de coupure en le modifiant chimiquement à chacune de ses extrémités par un groupement fluorophore quenché de type : Abz(NO₂)Tyr (extrémité-N : acide aminobenzoïque : extrémité-C : nitrotyrosine (amide)). L'hydrolyse de ce peptide par la protéase séparera le fluorophore et le quencheur et sera donc suivi par un accroissement de la fluorescence.

Un autre couple chimique tel que Dabcyl/EDANS peut être utilisé pour créer un autre type de substrat quenché.

On peut également envisager un substrat chromogénique en couplant le peptide avec le groupement paranitroanilide.

De la même façon, on peut envisager de développer un inhibiteur spécifique de la SASPase en remplaçant l'un des acides aminés d'un tel peptide, en modifiant la liaison peptidique ou en rajoutant un groupement chimique afin que la liaison soit rendue non hydrolysable par l'enzyme mais que le peptide ait toujours une affinité pour le site actif. Par exemple, on ajoute un acide aminé non naturel, on réduit la liaison peptide ou on ajoute des groupements chimiques de type aldéhyde, chlorométhyl-ketone ou diazométhyl-cétone.

Les inventeurs ont notamment montré que des modifications ponctuelles apportées au niveau du substrat peptidique figuré par SEQ ID NO : 30 pouvaient avoir un effet significatif au niveau de leur affinité pour l'enzyme.

Plus précisément, les séquences correspondantes modifiées s'avèrent susceptibles de constituer des inhibiteurs ou activateurs potentiels de la SASPase.

A titre illustratif de ces modulateurs potentiels, on peut plus particulièrement proposer des substrats peptidiques possédant au moins les séquences suivantes :

| | |
|---|---|
| EFDLELIEED | SEQ ID NO : 31 |
| EFDLDLIEED | SEQ ID NO : 32 |
| EFDLDLIEWD | SEQ ID NO : 33 |
| EFDLDLIHWD | SEQ ID NO : 34 |
| EPNLDLIEED | SEQ ID NO : 35 |

Il a en particulier été constaté un effet activateur des substrats représentés par les séquences SEQ ID NO : 31 et SEQ ID NO : 32.

La présente invention a également pour objet l'utilisation d'un polypeptide dont la séquence est représentée par une séquence choisie parmi SEQ ID NO : 31, SEQ ID NO : 32, SEQ ID NO : 33, SEQ ID NO : 34 et SEQ ID NO : 35, pour préparer une composition destinée à moduler l'activité de la protéase à acide aspartique de séquence SEQ ID NO : 5.

Les compositions pharmaceutiques ou cosmétiques revendiquées et considérées selon l'invention peuvent être des compositions utilisées dans les domaines cosmétique, dermatologique, dermato-cosmétique et pharmacologique.

Un milieu physiologiquement acceptable est selon l'invention un milieu cosmétiquement ou pharmaceutiquement acceptable compatible avec la peau, les muqueuses, les ongles et/ou les cheveux.

Les compositions selon l'invention peuvent être appliquées sur les ongles, les cheveux et plus particulièrement sur la peau et les muqueuses.

Elles sont particulièrement avantageuses pour agir sur un ou plusieurs mécanismes épidermiques tels que la dégradation de protéine(s), l'activation d'enzyme(s), et/ou la régulation du phénomène de différenciation/prolifération épidermique.

En l'occurrence, les compositions selon l'invention sont particulièrement utiles pour suppléer à un déséquilibre de la différenciation/prolifération épidermique. Plus particulièrement, elles peuvent être utiles pour réguler les phénomènes d'hydratation, d'inflammation, de mélanogénèse, et/ou de desquamation, le phénomène de vieillissement, les mécanismes de défense, pour la régulation de la différenciation/prolifération sur certains types cellulaires et cutanés : kératinocytes, mélanôcytes, cellules de Langerhans, sébocytes, adipocytes, ainsi que la régulation des phénomènes de sécrétion et de processus d'invasion.

D'une manière plus précise, les compositions revendiquées s'avèrent intéressantes dans les domaines suivants :
- pour traiter les affections dermatologiques liées à un désordre de la kératinisation portant sur la différenciation et sur la prolifération notamment pour traiter les acnés vulgaires, comédoniennes, polymorphes, rosacées, les acnés nodulokystiques, conglobata, les acnés séniles, les acnés secondaires telles que l'acné solaire, médicamenteuse ou professionnelle,
- pour traiter d'autres types de troubles de la kératinisation, notamment les ichtyoses, les état ichtyosiformes, la maladie de Darrier, les dératodermies palmoplantaires, les leucoplasies et les états leucoplasiformes, le lichen cutané ou muqueux (buccal),
- pour traiter d'autres affections dermatologiques liées à un trouble de la kératinisation avec une composante inflammatoire et/ou immuno-allergique et notamment toutes les formes de psoriasis qu'il soit cutané, muqueux ou unguéal, et même le rhumatisme psoriatique, ou encore l'atopie cutanée telle que l'eczéma, ou l'urticaire ou encore l'hypertrophie gingivale ; les composés peuvent également être utilisés dans certaines affections inflammatoires ne présentant pas de trouble de la kératinisation,
- pour traiter toutes les proliférations dermiques ou épidermiques qu'elles soient bénignes ou malignes, qu'elles soient ou non d'origine virale telles que verrues vulgaires, les verrues planes et l'épidermodysplasie verruciforme, les papillomatoses orales ou florides et les proliférations pouvant être induites par les ultraviolets notamment dans le cas des épithélioma baso- et spino-cellulaires,
- pour traiter d'autres désordres dermatologiques tels que les dermatoses bulleuses et les maladies du collagène,
- pour réparer ou lutter contre le vieillissement de la peau, qu'il soit photoinduit ou chronologique ou pour réduire les pigmentations et les kératoses actiniques,
ou toutes pathologies associées au vieillissement chronologique ou actinique,
- pour prévenir ou guérir les stigmates de l'atrophie épidermique et/ou dermique induite par les corticostéroïdes locaux ou systématiques, ou toute autre forme d'atrophie cutanée,
- pour prévenir ou traiter les troubles de la cicatrisation ou pour prévenir ou pour réparer les vergetures, et
- pour lutter contre les troubles de la fonction sébacée tels que l'hyperséborrhée de l'acné ou la séborrhée simple.

Dans le cas d'une application dans le domaine cosmétique, en particulier pour l'hygiène corporelle et capillaire, les compositions selon l'invention sont notamment utiles pour le traitement des peaux à tendance acnéique, pour la repousse des cheveux, l'antichute, pour lutter contre l'aspect gras de la peau ou des cheveux, dans la protection contre les aspects néfastes du soleil ou dans le traitement des peaux physiologiquement sèches, pour prévenir et/ou pour lutter contre le vieillissement photoinduit ou chronologique. Elles peuvent également être utiles pour améliorer les peaux reconstruites. On peut également utiliser directement dans le milieu de culture le polypeptide et/ou ses dérivés et/ou des modulateurs de son activité ou de son activation.

Un autre objet de l'invention est un procédé de traitement cosmétique destiné à lutter contre les troubles cutanés liés à un dysfonctionnement de la prolifération et/ou de la différenciation cellulaire comme notamment les peaux sèches, l'hyperkératose, la parakératose, les troubles de sébogénèse et/ou les signes de vieillissement cutané caractérisé en ce que l'on applique sur la peau, les muqueuses et/ou les fibres kératiniques une composition cosmétique comprenant au moins un polypeptide dont la séquence peptidique est une séquence choisie parmi SEQ ID NO : 6, SEQ ID NO : 16, SEQ ID NO : 25 et SEQ ID NO : 27 et leurs homologues possédant au moins 85 % d'homologie de séquence avec lesdites séquences et la même activité biologique

Le procédé de traitement de l'invention est un procédé cosmétique destiné à améliorer l'aspect esthétique de l'individu subissant des troubles de la prolifération et/ou de la différenciation épidermique.

L'invention concerne également l'utilisation d'un polypeptide dont la séquence peptidique est une séquence choisie parmi SEQ ID NO: 6, SEQ ID NO: 16, SEQ ID NO : 25 et SEQ ID NO: 27 et leurs homologues possédant au moins 85 % d'homologie de séquence avec lesdites séquences et la même activité biologique pour la préparation d'une composition pharmaceutique destinée au traitement des affections dermatologiques et notamment celles citées précédemment.

En particulier, l'invention concerne l'utilisation d'un polypeptide ou d'un mélange tel que décrit précédemment pour la préparation d'une composition pharmaceutique destinée à traiter l'ichtyose, le psoriasis ou toute pathologie impliquant une hyperkératose, une parakératose ou ayant une composante inflammatoire. Il peut également s'agir de compositions anti-douleur, de compositions pour traiter certaines maladies de la peau comme l'eczéma, la rosacée, le psoriasis, les lichens, les prurits sévères.

On sait qu'une protéine est synthétisée dans les cellules à partir d'une matrice d'acide désoxyribonucléique (ADN) codant pour ladite protéine. On sait également que le code génétique est dégénéré. Ainsi, la séquence d'acides aminés du polypeptide de l'invention peut être issue de différentes séquences d'acide désoxyribonucléique, naturelles ou synthétiques. Par séquence d'acide désoxyribonucléique synthétique, on entend ici toute séquence obtenue chimiquement ou par manipulation génétique.

Lesdites séquences d'acide désoxyribonucléique peuvent être issues de toutes origines possibles à savoir soit animale, en particulier de mammifères et encore plus particulièrement humaine, soit végétale, soit de micro-organismes (virus, phages, bactéries entre autres) ou encore de champignons, sans préjuger du fait qu'elles soient présentes de manière naturelle ou non dans ledit organisme d'origine.

En l'occurrence, l'invention se rapporte aux fragments d'acide désoxyribonucléique isolés et purifiés codant les polypeptides revendiqués.

Au cours de ces travaux, la demanderesse a pu isoler et purifier les fragments d'acide désoxyribonucléique codant les séquences primaires d'acides aminés des polypeptides de séquence SEQ ID NO : 5, SEQ ID NO: 6, SEQ ID NO: 7, SEQ ID NO : 16, SEQ ID NO : 25 ou SEQ ID NO : 27 à partir de peau humaine.

L'invention a pour objet un fragment d'acide désoxyribonucléique isolé et purifié dont la séquence nucléotidique comprend au moins la séquence nucléotidique codante SEQ ID NO : 24 et en particulier est représentée par la séquence nucléotidique codante SEQ ID NO : 20, SEQ ID NO : 24, SEQ ID NO : 26 ou SEQ ID NO : 28.

Les séquences d'acides nucléiques selon l'invention peuvent notamment être utilisées pour préparer des séquences d'acide ribonucléique correspondantes sens ou antisens.

L'invention a également pour objet tout polynucléotide, acide ribonucléique ou désoxyribonucléique, sens ou antisens, notamment « Small interferential RNA », correspondants au moins à la séquence nucléotidique codante SEQ ID NO : 20, SEQ ID NO : 24, SEQ ID NO : 26 ou SEQ ID NO : 28.

Les séquences d'acides nucléiques de l'invention peuvent également être utilisées pour réaliser des amorces oligonucléotidiques, qui s'hybrident dans des conditions de forte stringence à la séquence SEQ ID NO : 17, SEQ ID NO : 18, SEQ ID NO : 19, SEQ ID NO : 20, SEQ ID NO : 21, SEQ ID NO : 22, SEQ ID NO : 23, SEQ ID NO : 24, SEQ ID NO : 26 et SEQ ID NO : 28.

Ces amorces oligonucléotidiques sens et/ou antisens peuvent être utiles pour des réactions de séquençage ou d'amplification spécifique selon la technique dite de PCR (réaction de polymérisation en chaîne) ou toute autre variante de celle-ci dans le but de clonage, d'identification ou de diagnostic d'un polypeptide représenté par SEQ ID NO : 1, SEQ ID NO : 4, SEQ ID NO : 5, SEQ ID NO: 6, SEQ ID NO : 7, SEQ ID NO : 8, SEQ ID NO : 9, SEQ ID NO : 16, SEQ ID NO : 25 ou SEQ ID NO : 27.

En particulier, les sondes ou amorces de l'invention sont marquées, préalablement à leur utilisation. Pour cela, plusieurs techniques sont à la portée de l'homme du métier comme par exemple le marquage fluorescent, radioactif, chimioluminescent ou enzymatique.

Les méthodes de diagnostic in vitro dans lesquelles ces sondes nucléotidiques sont mises en oeuvre pour la détection de synthèses de séquences nucléiques codant pour un polypeptide selon l'invention sont incluses dans la présente invention.

Le fragment d'ADN correspondant à la séquence SEQ ID NO : 19, SEQ ID NO : 20, SEQ ID NO : 24, SEQ ID NO : 26 ou SEQ ID NO : 28 peut également être introduit dans un vecteur d'expression permettant ainsi la synthèse d'une protéine dite recombinante correspondante.

L'invention a donc également pour objet un vecteur d'expression recombinant contenant tout ou partie de la séquence nucléotidique codante SEQ ID NO : 20, SEQ ID NO : 24, SEQ ID NO : 26 ou SEQ ID NO : 28.

L'invention a également pour objet une composition cosmétique ou pharmaceutique comprenant, dans un milieu physiologiquement acceptable une séquence d'acide désoxyribonucléique, naturelle ou synthétique, codant pour la séquence primaire d'acides aminés d'un polypeptide conforme à l'invention ou une séquence d'acide ribonucléique sens ou antisens, notamment antisens interférentiel, correspondants à ladite séquence SEQ ID NO : 20, SEQ ID NO : 24, SEQ ID NO : 26 ou SEQ ID NO : 28.

D'une manière générale, toute composition de l'invention peut être ingérée, injectée ou appliquée sur la peau (sur toute zone cutanée du corps) ou sur les muqueuses (buccale, jugale, gingivale, génitale, conjonctivale, ...).

De préférence, une composition de l'invention est appliquée sur la peau ou les muqueuses.

Selon le mode d'administration considéré, elle peut se présenter sous toutes les formes galéniques normalement utilisées.

Pour une application topique sur la peau, la composition peut avoir la forme notamment de solutions aqueuses ou huileuses ou de dispersions du type lotion ou sérum, d'émulsions de consistance liquide ou semi-liquide du type lait, obtenues par dispersion d'une phase grasse dans une phase aqueuse (H/E) ou inversement (E/H), ou de suspensions ou émulsions de consistance molle du type crème ou gel aqueux ou anhydres, ou encore de microcapsules ou microparticules, ou de dispersions vésiculaires de type ionique et/ou non ionique ou de mousses. Ces compositions sont préparées selon les méthodes usuelles.

Pour l'injection, la composition peut se présenter sous forme de lotions aqueuses, huileuses ou sous forme de sérums. Pour les yeux, elle peut se présenter sous forme de gouttes et pour l'ingestion, elle peut se présenter sous forme de capsules, de granulés, de sirops ou de comprimés.

Les quantités des différents constituants des compositions selon l'invention sont celles classiquement utilisées dans les domaines considérés.

Dans le domaine de la cosmétique, ces compositions constituent notamment des crèmes de nettoyage, de protection, de traitement ou de soin pour le visage, pour les mains, pour les pieds, pour les grands plis anatomiques ou pour le corps (par exemple crèmes de jour, crèmes de nuit, crèmes de démaquillage, crèmes de fond de teint, crèmes anti-solaires), des fonds de teint fluides, des laits de démaquillage, des laits corporels de protection ou de soin, des laits anti-solaires, des lotions, gels ou mousses pour le soin de la peau, comme des lotions de nettoyage, des lotions anti-solaires, des lotions de bronzage artificiel, des compositions pour le bain, des compositions désodorisantes comprenant un agent bactéricide, des gels ou lotions après-rasage, des crèmes épilatoires, des compositions contre les piqûres d'insectes, des compositions anti-douleur, des compositions pour traiter certaines maladies de la peau comme l'eczéma, la rosasée, le psoriasis, les lichens et les prurits sévères.

Les compositions selon l'invention peuvent également consister en des préparations solides constituant des savons ou des pains de nettoyage.

Les compositions peuvent aussi être conditionnées sous forme de composition pour aérosol comprenant également un agent propulseur sous pression.

Une composition selon l'invention peut aussi être une composition pour les soins du cuir chevelu, et notamment un shampoing, une lotion de mise en plis, une lotion traitante, une crème ou un gel coiffant, une composition de teintures (notamment teintures d'oxydation) éventuellement sous forme de shampoings colorants, de lotions restructurantes pour les cheveux, une composition de permanente (notamment une composition pour le premier temps d'une permanente), une lotion ou un gel antichute, un shampoing antiparasitaire, antipelliculaire etc.

Une composition peut aussi être à usage bucco-dentaire, par exemple une pâte dentifrice. Dans ce cas, la composition peut contenir des adjuvants et additifs usuels pour les compositions à usage buccal et notamment des agents tensioactifs, des agents épaississants, des agents humectants, des agents de polissage tels que la silice, divers ingrédients actifs comme les fluorures, en particulier le fluorure de sodium, et éventuellement des agents édulcorants comme le saccharinate de sodium.

Lorsque la composition est une émulsion, la proportion de la phase grasse peut varier d'environ 5 % à 80 % en poids, et de préférence d'environ 5 % à 50 % en poids par rapport au poids total de la composition. Les huiles, les cires, les émulsionnants et les co-émulsionnants utilisés dans la composition sous forme d'émulsion sont choisis parmi ceux classiquement utilisés dans le domaine cosmétique. L'émulsionnant et le co-émulsionnant sont présents, dans la composition, en une proportion allant de 0,3 % à 30 % en poids, et de préférence de 0,5 % à 20 % en poids par rapport au poids total de la composition. L'émulsion peut, en outre, contenir des vésicules lipidiques.

Lorsque la composition est une solution ou un gel huileux, la phase grasse peut représenter plus de 90 % du poids total de la composition.

De façon connue, la composition cosmétique peut contenir également des adjuvants habituels dans le domaine cosmétique, tels que les gélifiants hydrophiles ou lipophiles, les additifs hydrophiles ou lipophiles, les conservateurs, les antioxydants, les solvants, les parfums, les charges, les filtres, les absorbeurs d'odeurs et les matières colorantes. Les quantités de ces différents adjuvants sont celles classiquement utilisées dans le domaine cosmétique, et par exemple varient d'environ 0,01 % à 10 % du poids total de la composition. Ces adjuvants, selon leur nature, peuvent être introduits dans la phase grasse, dans la phase aqueuse et/ou dans les sphérules lipidiques.

Comme huiles ou cires utilisables dans l'invention, on peut citer les huiles minérales (huile de vaseline), les huiles végétales (fraction liquide du beurre de karité, huile de tournesol), les huiles animales (perhydrosqualène), les huiles de synthèse (huile de Purcellin), les huiles ou cires siliconées (cyclométhicone) et les huiles fluorées (perfluoropolyéthers), les cires d'abeille, de carnauba ou paraffine. On peut ajouter à ces huiles des alcools gras et des acides gras (acide stéarique). Comme émulsionnants utilisables dans l'invention, on peut citer par exemple le stéarate de glycérol, le polysorbate 60 et le mélange de PEG-6/PEG-32/Glycol Stéarate vendu sous la dénomination de Tefose^{®} 63 par la société Gattefosse.

Comme solvants utilisables dans l'invention, on peut citer les alcools inférieurs notamment l'éthanol et l'isopropanol et le propylène glycol.

Comme gélifiants hydrophiles utilisables dans l'invention, on peut citer les polymères carboxyvinyliques (carbomer^{®}), les copolymères acryliques tels que les copolymères d'acrylates/alkylacrylates, les polyacrylamides, les polysaccharides tels que l'hydroxypropylcellulose, les gommes naturelles et les argiles, et, comme gélifiants lipophiles, on peut citer les argiles modifiées comme les bentones, les sels métalliques d'acides gras comme les stéarates d'aluminium, la silice hydrophobe, l'éthylcellulose et le polyéthylène.

La composition peut contenir d'autres actifs hydrophiles comme les protéines ou les hydrolysats de protéines, les acides aminés, les polyols, l'urée, l'allantoïne, les sucres et les dérivés de sucre, les vitamines hydrosolubles, les extraits végétaux et les hydroxyacides.

Comme actifs lipophiles, on peut utiliser le rétinol (vitamine A) et ses dérivés, le tocophérol (vitamine E) et ses dérivés, les acides gras essentiels, les céramides, les huiles essentielles, l'acide salicylique et ses dérivés.

Selon l'invention la composition peut associer au moins un autre agent actif destiné notamment à la prévention et/ou au traitement des affections cutanées. Parmi ces agents actifs, on peut citer à titre d'exemple :
- les agents diminuant la différenciation et/ou la prolifération et/ou la pigmentation cutanée tels que l'acide rétinoïque et ses isomères, le rétinol et ses esters, la vitamine D et ses dérivés, les oestrogènes tels que l'oestradiol, l'acide kojique ou l'hydroquinone ;
- les antibactériens tels que le phosphate de clindamycine, l'érythromycine ou les antibiotiques de la classe des tétracyclines ;
- les antiparasitaires, en particulier le métronidazole, le crotamiton ou les pyréthrinoïdes ;
- les antifongiques, en particulier les composés appartenant à la classe des imidazoles tels que l'éconazole, kétoconazole ou le miconazole ou leurs sels, les composés polyènes, tels que l'amphotéricine B, les composés de la famille des allylamines, tels que la terbinafine, ou encore l'octopirox ;
- les agents antiviraux tels que l'acyclovir ;
- les agents anti-inflammatoires stéroïdiens, tels que l'hydrocortisone, le valérate de bétaméthasone ou le propionate de clobétasol, ou les agents anti-inflammatoires non-stéroïdiens comme par exemple l'ibuprofène et ses sels, le diclofénac et ses sels, l'acide acétylsalicylique, l'acétaminophène ou l'acide glycyrrhizique ;
- les agents anesthésiques tels que le chlorhydrate de lidocaïne et ses dérivés ;
- les agents antiprurigineux comme la thénaldine, la triméprazine ou la cyproheptadine ;
- les agents kératolytiques tels que les acides α- et β-hydroxycarboxyliques ou β-cétocarboxyliques, leurs sels, amides ou esters et plus particulièrement les hydroxyacides tels que l'acide glycolique, l'acide lactique, l'acide salicylique, l'acide citrique et de manière générale les acides de fruits, et l'acide n-octanoyl-5-salicylique ;
- les agents anti-radicaux libres, tels que l'α-tocophérol ou ses esters, les superoxydes dismutases, certains chélatants de métaux ou l'acide ascorbique et ses esters ;
- les antiséborrhéiques tels que la progestérone ;
- les antipelliculaires comme l'octopirox ou la pyrithione de zinc ;
- les antiacnéiques comme l'acide rétinoïque ou le peroxyde de benzoyle.

Ainsi, selon le mode particulier, la composition selon l'invention comprend également au moins un agent choisi parmi les agents antibactériens, antiparasitaires, antifongiques, antiviraux, anti-inflammatoires, antiprurigineux, anesthésiques, kératolytiques, anti-radicaux libres, anti-séborrhéiques, antipelliculaires, antiacnéiques et/ou les agents diminuant la différenciation et/ou la prolifération et/ou la pigmentation cutanée.

Les exemples figurant ci-après sont présentés à titre illustratif et non limitatif de l'invention.

### FIGURES

- Figure 1 : Photographie du gel d'électrophorèse 2D obtenu selon l'exemple I,
- Figure 2 : Représentation de l'influence du pH sur l'activité protéolytique de la SASPase,
- Figure 3 : Analyse de l'activité d'un certain nombre de modulateurs conventionnels vis-à-vis de l'activité protéolytique de la SASPase,

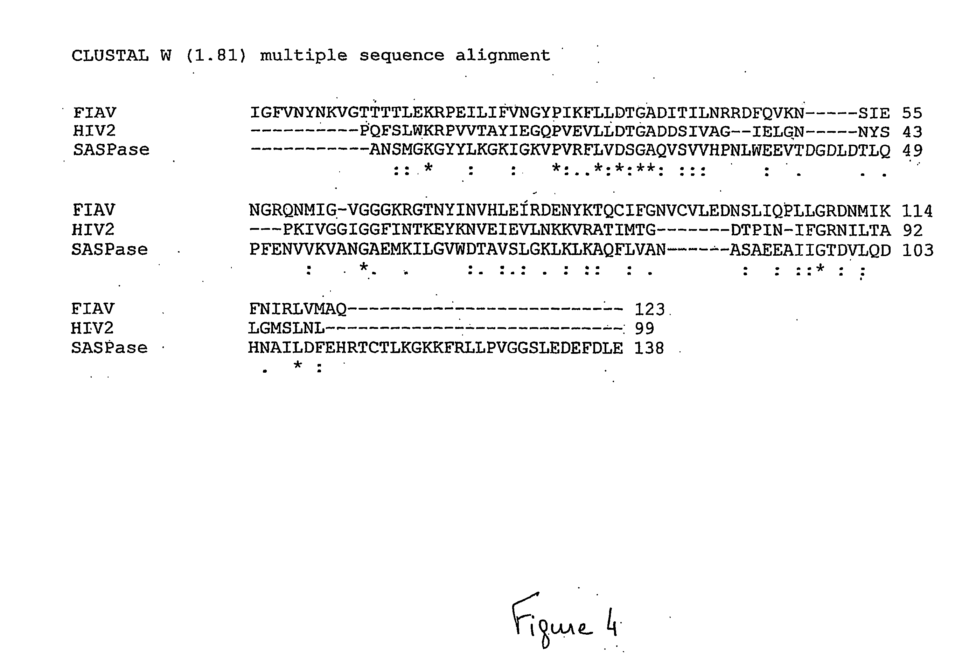

- Figure 4 : Représentation des homologies de structure entre la SASPase et des protéases,
- Figure 5 : Représentation de l'hydrolyse des substrats représentés par les séquences SEQ ID NO : 31, SEQ ID NO : 32, SEQ ID NO : 33, SEQ ID NO : 34 et SEQ ID NO : 35 par la SASPase.

### EXEMPLES

### Matériels

Les oligonucléotides utilisés dans les différentes expériences sont présentés ci-après dans le tableau I.

**TABLEAU I**

| Nom de l'amorce | Séquence 5' à 3' | SEQ ID N° |
|---|---|---|
| SC 130 | GATAGGATCCATGGCCGGGAGCGGAGCCAGGAG | 12 |
| SC 131 | TTGAATTCTCAGTGGGATAGCTCCTGCCGC | 11 |
| SC 134 | GGCCCTGGGTGTCTACAATA | 13 |
| SC 135 | TTGGCCACCTTTACCACATT | 14 |
| SC 140 | TAGGATCCATGGGGAGCCCAGGGGC | 10 |
| Not I-(dT)₁₈ est un oligonucléotide vendu par la société Amersham Pharmacia Biotech | | |

| | | |
|---|---|---|
| Not I-(dT)₁₈ est conçu pour se fixer aux longues séries de A, par exemple les poly A +. Utilisé dans les réactions de transcription inverse, Not I-(dT)₁₈ se fixe au poly A +. | | |

### EXEMPLE I - Identification et isolement de la SASPase par électrophorèse bidimensionnelle sur gel et séquençage

### a) Préparation à partir d'épiderme humain

Tampons :
I : SDS 0,3 % ; tris-HCl 28 mM ; tris-base 22 mM
2D : 8M urée ; 2 % (p/v) 3-[(3-cholamidopropyl)diméthylammonio]-1-propane-sulfonate (CHAPS) ; Dithiothréitol 20 mM ; tampon 0,5 % (v/v) Imobiline pH gradient (IPG) pH = 3 9-10 de 18 cm commercialisé par la société Amersham Pharmacia Biotech.

Des extraits d'épiderme reconstruit humain sont préparés à partir de 30 nacelles du kit Episkin J13. 15 ml de tampon I sont ajoutés aux 30 épidermes reconstruits et le tout est pottérisé, porté à ébullition pendant 10 mn puis repottérisé. La solution est alors centrifugée à 10.000 g pendant 10 mn. Le surnageant est recueilli et filtré sur membrane 0,22 µm. On obtient ainsi 12 ml de surnageant SI. On ajoute alors au surnageant SI, de l'acétone froide (10 v/2v). Après 20 mn d'incubation, on centrifuge le mélange obtenu à 9.400 g pendant 10 mn. Le surnageant est alors éliminé et le culot est séché à température ambiante pendant 20 mn. Le culot est alors repris dans 2 ml de tampon 2D. On obtient ainsi l'extrait EI. La concentration en protéine finale est de 11 mg/ml.

### b) Gel bidimensionnel

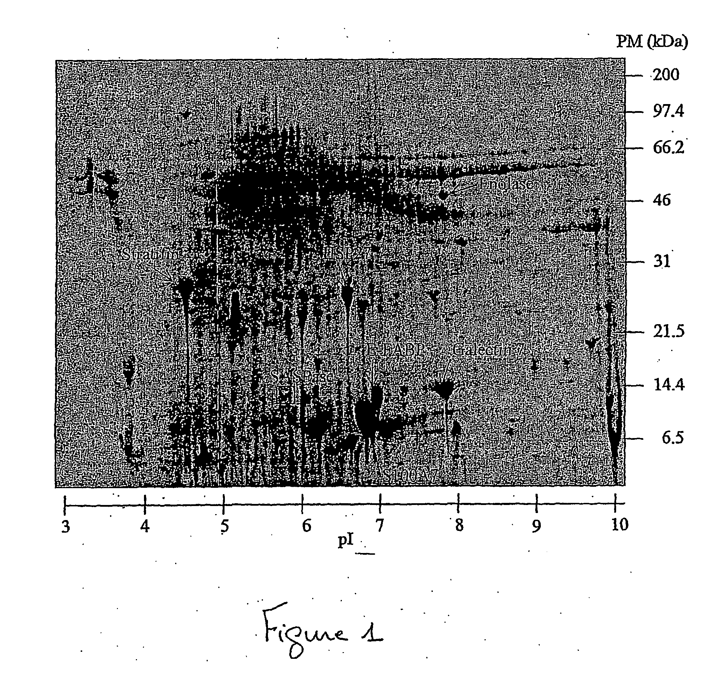

La séparation en deux dimensions des protéines contenues dans l'extrait EI est réalisée sur un appareil de marque Pharmacia (modèle Multiphor II). La séparation en deux dimensions des protéines a été réalisée selon les recommandations du fournisseur hormis le fait que pour la rééquilibration du gel IPG après migration dans la première direction, l'iodoacétamide a été omis. La coloration des spots, la récupération de ceux-ci et le séquençage des polypeptides qu'ils contenaient ont été réalisés selon les techniques décrites dans Méhul B, Bernard D, Simonetti L, Bernard MA, Schmidt R : Identification and cloning of a new calmodulin-like protein from human epidermis. J Biol Chem 275 : 12841-12347, 2000, ou encore « A practical guide to protein and peptide purification for microsequencing » (Paul Matsudaira éditeur, seconde édition 1993). En figure 1 est représenté le gel d'électrophorèse correspondant.

Les protéines ont été détectées par une coloration à l'amide black. Les spots correspondant à des protéines identifiées par séquençage d'Edman sont localisés avec le nom de ces protéines. Le spot nommé SASPase permet de localiser une forme épidermique de la protéine ayant un PM apparent de 12 kD et un pI de 5,8.

Les résultats obtenus ont permis de caractériser les séquences SEQ ID NO : 1, SEQ ID NO : 2 et SEQ ID NO : 3.

### EXEMPLE II - Isolement de l'ADNc codant la SASPase à partir de kératinocyte humain et expression de la SASPase (SEQ ID NO : 5) et de sa forme tronquée (Δ 1-84) (SEQ ID NO : 4)

### a) Préparation de L'ADNc

Les ARN totaux de kératinocytes provenant d'épiderme humain reconstruit après 13 jours de culture ont été préparés à l'aide du kit de préparation d'ARN « RNeasy Kit^{®} » et purifiés à l'aide du kit « QIAshredder column^{®} », commercialisé par la société QIAGEN selon les instructions du fournisseur.

Les ADN complémentaires (ADNc) des ARN ainsi préparés ont été synthétisés à l'aide du kit « First Strand cDNA Synthesis^{®} » commercialisé par la société Amersham Pharmacia Biotech selon les instructions du fournisseur en utilisant comme amorce, l'oligonucléotide Not I-(dT)₁₈.

Des fragments d'ADNc codant pour la SASPase complète ainsi obtenus ont été amplifiés par des réactions de polymérisation en chaîne (PCR) dans un appareil à cycles thermiques « Thermocycler^{®} » commercialisé par la société Perkin-Elmer en utilisant une ADN polymérase pfu commercialisée par la société Promega et comme amorces, le couple d'oligonucléotides SC140 (SEQ ID NO : 10)/SC131 (SEQ ID NO : 11) et les conditions suivantes : 1 cycle (95°C pendant 2 mn.), 35 cycles (94°C pendant 30 sec., 65°C pendant 30 sec., 72°C pendant 2 mn.) et 1 cycle (72°C pendant 7 mn.).

De façon similaire, des fragments d'ADNc codant pour la forme tronquée de la SASPase (SEQ ID NO : 4), dépourvue des 84 résidus d'acides aminés N-terminaux de la SASPase dite Δ 1-84 ont été amplifiés par PCR en utilisant comme amorces, le couple d'oligonucléotides SC131/SC130 (SEQ ID NO : 11 et SEQ ID NO : 12).

### b) Construction et expression de la SASPase recombinante (SASPase)

L'ADNc de la SASPase obtenu précédemment est introduit dans le vecteur plasmidique pGex-4T-3 commercialisé par la société Amersham Pharmacia Biotech, par coupure/ligature aux sites de restriction BamH-1/EcoR1. Ce plasmide recombinant qui contient en phase dans le cadre de lecture, la séquence codante de la SASPase (SEQ ID NO : 5) et la séquence codante de la glutathion S-transférase (GST) est alors introduit dans *E. coli* souche *BL21* (DE3) commercialisé par la société Amerscham Pharmacia Biotech. La protéine de fusion recombinante exprimée par les bactéries peut être coupée par la thrombine dans des conditions douces, la construction étant telle que la protéine de fusion obtenue porte un site de coupure par cette protéase.

Le produit d'expression est purifié par chromatographie d'affinité sur colonne gluthation-sépharose.

L'ensemble de ces expériences a été réalisé en appliquant strictement les différents protocoles des fournisseurs.

L'analyse par électrophorèse sur gel SDS-PAGE d'une fraction aliquote du produit d'expression obtenu par mise en oeuvre de la méthode décrite précédemment montre que cette méthode permet l'obtention en quantité satisfaisante de la protéine de fusion recombinante GST-SASPase.

De façon similaire, on a obtenu l'expression en quantité satisfaisante de la protéine de fusion recombinante GST-SASPase tronquée Δ 1-84 (SEQ ID NO : 4).

### EXEMPLE III - Obtention de la SASPase activée (SEQ ID NO 6)

La protéine de fusion recombinante GST-SASPase Δ 1-84 (SEQ ID NO : 4) éluée de la colonne glutathion sépharose obtenue à l'exemple II, est incubée avec de la thrombine dans du tampon PBS, à pH8 pendant 18 heures à 22°C. Le tampon est échangé par filtration sur gel G25^{®} Biorad contre une solution 100 mM acétate, pH 4,5. Le mélange ainsi obtenu est soumis à une chromatographie cationique en utilisant un gradient de NaCl (0 à 1 M NaCl). Les fractions contenant la GST et la thrombine sont éliminées et la fraction éluée par la solution à 750 mM de NaCl est récupérée. Une analyse par électrophorèse sur gel SDS-PAGE effectuée sur la fraction éluée par la solution de NaCl à 750 mM, contenant une activité caséinolytique (donnée non présentée) montre la présence d'une bande majoritaire qui, par comparaison avec les marqueurs de masses moléculaires présente une masse moléculaire apparente de 12kD et une bande minoritaire qui correspond à la forme tronquée SASPase Δ1-84 (SEQ ID NO : 4).

La forme de 12kD correspond à la forme activée de la SASPase. Le séquençage d'Edman indique que le produit isolé correspond à la SASPase activée (SEQ ID NO : 6).

### EXEMPLE IV - Obtention de la SASPase activée (SEQ ID NO : 25)

La délétion du site codant pour la séquence FANS sur la forme tronquée de la SASPase (SEQ ID NO : 4) à l'aide du kit Quick Change Site-directed Mutagenesis (Stratagene) et des primers adaptés est effectuée en utilisant comme matrice d'ADN la construction plasmidique de la séquence de la SASPase C27 (SEQ ID NO : 4) dans le vecteur pGEX-4T3.

Une protéine dont la séquence protéique est représentée par SEQ ID NO : 36 est obtenue.

Après incubation à pH 5,00 dans du tampon acétate de cette nouvelle protéine il y a, de façon surprenante, génération d'un fragment de bas poids moléculaire (environ 21 kDa en électrophorèse SDS-PAGE) qui possède au plus la séquence SEQ ID NO : 25 de masse théorique 18731.**44**.

La SEQ ID NO : 27 de masse théorique 16794.43 est obtenue à partir de la SEQ ID NO : 25 en assumant une activation en C-terminal identique à la forme SEQ ID NO : 6.

### EXEMPLE V - Caractérisation de l'activité protéolytique de la protéine SASPase

### a) Activité vis-à-vis d'un substrat

Cette activité a été démontrée selon le protocole suivant :

L'insuline chaine Beta oxydée (Sigma) est utilisée comme substrat. La concentration est de 50µg dans 1,5 ml de tampon acétate 0,1M pH 5,0. On ajoute 50 µl de SASPase 12 kD purifiée (SEQ ID NO : 6) (environ 1mg/ml) par gel filtration pour initialiser l'hydrolyse. Des témoins sans enzyme ou sans substrat sont utilisés comme contrôles. Au cours du temps (lh à 24h à 37° C) des HPLC sont effectuées pour suivre l'hydrolyse. Les pics apparaissant rapidement correspondent aux sites d'hydrolyse principaux et ceux n'apparaissant qu'au bout de 24h aux sites secondaires. Les pics sont collectés après leur fractionnement et séquencés en N-terminal (séquençage d'EDMAN, Institut Pasteur).
FVNQHLCGSHLVEALYLVCGERGFF (SEQ ID NO : 15).
Site principal : E/A (de type pepsin-like)
Sites secondaires : L/Y et Y/L (de type pepsin-like)

Ces résultats montrent que la SASPase activée est capable de dégrader l'insuline. La dénaturation thermique (95° C - 10 mn) de la SASPase abolit son activité de dégradation de l'insuline. Son activité caséinolytique a par ailleurs été démontrée (résultats non présentés).

### b) Activité autocalytique

Dans ce second essai, c'est la protéine GST-SASPase (SEQ ID NO : 4) qui est elle-même utilisée comme substrat par autocatalyse.

La GST-SASPase à 3mg/ml dans du tampon phosphate 0,1M pH 7,0 50 % glycérol est acidifiée rapidement à pH 5,0 par du tampon acétate 1M pH 4,5. A chaque temps d'incubation à 37° C un aliquot est pris et la réaction est bloquée par l'ajout d'un équivalent volume de tampon Laemmli sans DTT. A la fin de la cinétique chaque échantillon est analysé par électrophorèse SDS-PAGE (Gel à 15 % d'acrylamide) démontrant une apparition progressive d'une bande majoritaire migrant à un PM apparent de 12 kD consécutive à la disparition de la protéine de fusion.

Cet essai montre que l'on peut également obtenir directement la SASPase activée (SEQ ID NO : 6) par acidification à pH 3 à 6, de préférence 4 à 6 de la protéine de fusion recombinante GST-rSASPase Δ1-84 ou GST-SASPase obtenues à l'exemple I suivi par une étape de purification par filtration sur gel (G75).

Par ailleurs, l'analyse des solutions activées par séquençage Edman et par spectromètre de masse de type QTOF fournit pour la protéase autoactivée trois sites de clivage à peu près équivalents en probabilités :
F/A (comme certaines cutané métalloprotéases)
N/S (site de coupure non répertorié)
E/L (uniquement décrit pour la matrilysine qui est capable d'activer l'urokinase et les MMP 1, 2 et 9).

### EXEMPLE VI - Mutations dirigées sur le site actif de la SASPase prouvant son appartenance à la famille des protéases à acide aspartique.

Le kit Quick Change Site-directed Mutagenesis (Stratagene) est utilisé pour la réalisation des mutants de la SASPase.

La séquence de la SASPase SEQ ID NO : 5 est modifiée au niveau de l'acide aspartique n° 212, soit son site actif potentiel. Pour ce faire, deux oligonucléotides sont synthétisés de telle sorte que l'acide aminé 212 soit substitué soit en alanine (mutant A/D), soit en acide glutamique (mutant E/D).

Une amplification par PCR est réalisée avec chaque couple d'oligonucléotides mutés, la construction plasmidique de la séquence de la SASPase C27 dans le vecteur pGEX-4T3 est utilisée comme matrice d'ADN.

Des bactéries compétentes (XL1 blue) sont ensuite transformées avec chaque produit de PCR. Plusieurs clones sont amplifiés et séquencés afin de vérifier la présence de la mutation souhaitée et seulement de celle-ci.

### Autoclivage de la SASPase C27 et de ses mutants :

Une production des protéines recombinantes mutées est réalisée selon le même protocole que pour la forme sauvage, SASPase C27; **(SEQ ID NO : 4).**

Chaque protéine recombinante produite sous forme de protéine de fusion est ensuite incubée dans un tampon acétate 1M, pH 4.5 à 37 °C. Des prélèvements au cours du temps sont réalisés et analysés par SDS-PAGE.

On observe une diminution au cours du temps de la forme entière GST-SASPase C27, ce qui démontre un autoclivage et par conséquent une autoactivation ; de nombreuses bandes de poids moléculaires inférieurs apparaissent. Les formes mutées (A/D) et (E/D), quant à elles, ne s'autoactivent pas.

Cette expérience confirme l'implication de l'acide aspartique n° 212 dans le site actif de la SASPase.

### EXEMPLE VII - Analyse de l'expression de la SASPase par Northern Blot, RT-PCR dans des tissus humains et dans des kératinocytes.

Les analyses ont été effectuées par Northern Blot, en utilisant des membranes commerciales polyA + ARN blot (Ambion^{®}) selon le protocole décrit par le fabricant, et par RT-PCR en utilisant une collection d'ADNc des membranes « rapid-scan^{®} » humaines commercialisées par la société OriGene Technologies, Inc.

L'analyse par Northem Blot a été effectuée en utilisant comme sonde l'ADNc codant la forme (tronquée Δ1-84) de la SASPase (SEQ ID NO : 4) isolée à partir du plasmide recombinant préparé à l'exemple II et à l'aide d'une membrane contenant différents échantillons d'ARN.

La PCR est effectuée en utilisant la Taq polymérase commercialisée par la société Promega, comme amorces, le couple d'oligonucléotides SC134 (SEQ ID NO : 13)/SC135 (SEQ ID NO : 14) dans les conditions suivantes :
- 1 cycle de 3 mn. à 94°C,
- 25 à 30 cycles (94°C pendant 30 sec., 53°C pendant 30 sec., 72°C pendant 60 sec.), et
- 1 cycle à 72°C pendant 5 mn.
   Les produits de PCR résultants sont visualisés par coloration au bromure d'éthidium après séparation sur gel d'agarose 2 % (poids/volume).
   Des résultats, il ressort que la SASPase est sensiblement exprimée dans la quasi-totalité des tissus testés à un niveau faible dans les foie foetal, cerveau foetal, ovaire, glande surrénale, thyroïde, placenta, testicules, estomac, muscle, poumon, foie, rate et coeur, et encore plus faible dans la moelle osseuse, le pancréas, la salive, et l'intestin grêle. Cette expression est en revanche significative dans le cerveau et particulièrement très élevée dans la peau.

### EXEMPLE VIII - Oligomérisation de la SASPase utilisant un réactif de réticulation

Le protocole utilisé s'inspire de la technique décrite dans T.D. Meek et al. ; Proc, Natl. Acad. Sci. USA vol. 86, pages 1841-1845, March 1989.

Une étude de la réticulation utilisant du BS3 (Pierce) est effectuée sur de la SASPase activée (SEQ ID NO : 6), obtenue par acidification et purification par chromatographie d'exclusion (gel filtration) de la protéine de fusion recombinante GST-SASPase Δ1-84.

A la SASPase activée (SEQ ID NO : 6) obtenue à l'exemple III, placée dans de la glace, on ajoute 1 µg de BS3 dans 60 µl de tampon phosphate 50 mM, pH 7, contenant 150 mM NaCl, 0,1 % TX100, 5 mM EDTA. On prépare également un échantillon témoin exempt de BS3. Après 90 mn, on introduit dans les milieux réactionnels 2 µl de Tris-HCl 1 M, pH 8, pour stopper la réaction. On analyse ensuite les produits obtenus par gel filtration sur gel, et par électrophorèse SDS-PAGE.

La SASPase activée incubée avec du BS3 forme un complexe multimérique stable qui par gel filtration sur gel, se sépare en trois pics majeurs. Trois bandes distinctes sont également observables sur gel après électrophorèse SDS-PAGE dont les masses moléculaires apparentes, déterminées par comparaison avec les marqueurs des masses moléculaires sont respectivement de 12kD, comprises entre 10-14kD, 30-45kD et 60-100kD.

### EXEMPLE IX - Influence du pH sur l'activité protéolytique de SASPase

10 µl de protéines de fusion GST-SASPase Δ 1-84 (environ 3 mg/ml) obtenus à l'exemple II sont incubés dans 200 µl de tampon acétate 0,1 M, ajusté à différents pH en présence du substrat caséine commercialisé sous la dénomination de Enzchek ^{®} par la société Molecular Probes et utilisé aux concentrations préconisées par le fournisseur.

Trois répétitions sont effectuées pour chaque essai.

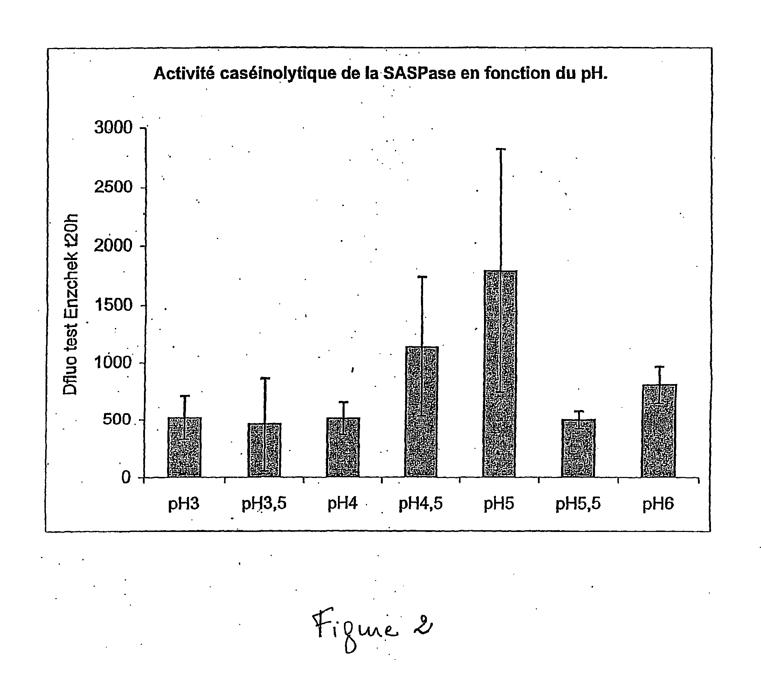

Après 20 heures d'incubation à 37°C, la fluorescence est mesurée sur un lecteur de plaque commercialisé sous la dénomination de Biolumin ^{®} par la société Molecular Probes en utilisant le couple excitation/émission : 485/535 nm. Les résultats sont présentés en figure 2.

Ces résultats montrent que dans les conditions expérimentales retenues, l'activité enzymatique de la SASPase est dépendante du pH et présente un optimum à pH 5. Par ailleurs, des essais non présentés en tampon phosphate 0,1 M de pH 6 à 7,5 ne montrent qu'une faible activité résiduelle.

L'influence du pH sur l'auto-activation est également réalisée et montre une optimisation pour des pH entre 3 et 6,9 et de préférence entre 4 et 6.

### EXEMPLE X - Etude de l'effet de différents inhibiteurs de la famille des protéases à acide aspartique sur l'activité caséinolytique de la SASPase activée (SEQ ID NO : 6)

10 µl de SASPase activée (SEQ ID NO : 6) auto-activés (environ 3 mg/ml), sont ajoutés dans 200 µl de tampon acétate 0,1 M, pH 5,5, contenant 20 µl de DMSO avec des inhibiteurs de rétropepsines. Un essai témoin est effectué dans les mêmes conditions en absence d'inhibiteur.

On pré-incube le mélange pendant 1 heure à 4°C, puis on ajoute le substrat, de la caséine commercialisée sous la dénomination Enzchzek ^{®} par la société Molecular Probes en quantité suffisante pour obtenir la concentration préconisée par le fournisseur, les solutions ayant été préalablement réchauffées à 37°C.

Le suivi de l'hydrolyse de la caséine est effectué par mesure de la fluorescence sur un lecteur de plaques Biolumin ^{®} commercialisé par la société Molecular Probes en utilisant le couple excitation/émission : 485/535 nm.

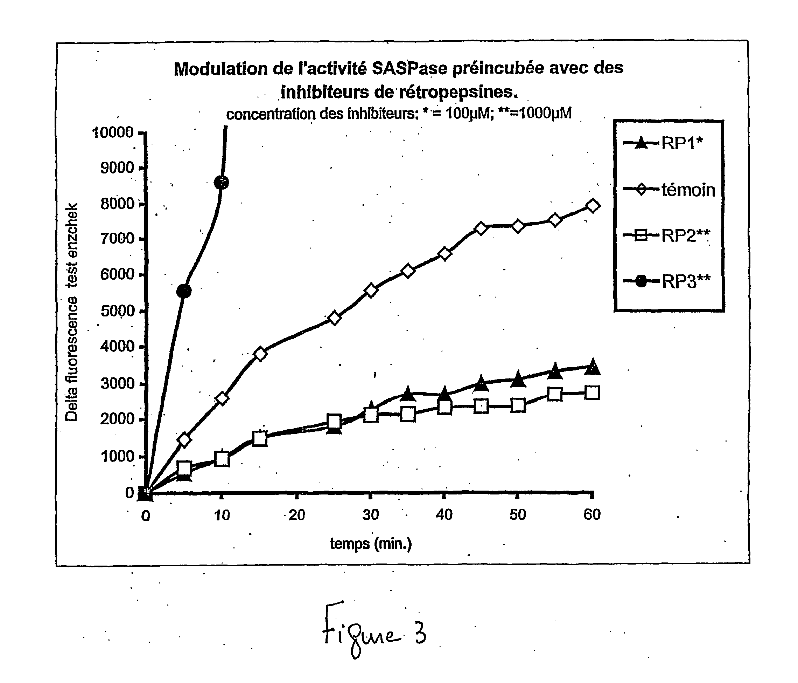

Les résultats obtenus sont présentés en figure 3. On constate que les cinétiques d'hydrolyse de la caséine, en présence des inhibiteurs de rétropepsines RP1 et RP2 sont plus faibles que le témoin. L'activité de la SASPase est donc inhibée par ces inhibiteurs.
- RP1 correspond à la séquence Ac-Leu-Val-Phe-aldéhyde et est commercialisé par Bachem sous la référence N 1395.0005,
- RP2 correspond à la séquence Ac-Leu-Leu-Met-aldéhyde et est commercialisé par Bachem sous la référence N 1315.0005 et
- RP3 correspond à la séquence Ac-Leu-Leu-Nle-aldéhyde et est commercialisé par Bachem sous la référence N 1320.0005.

On constate en outre que la cinétique d'hydrolyse de la caséine, en présence de l'inhibiteur de rétropepsine RP3, est significativement plus élevée que celle obtenue dans les conditions témoins. L'activité de la SASPase semble donc être stimulée par cet inhibiteur.

### EXEMPLE XI - Etude de l'effet de la SASPase activée (SEQ ID NO : 6) sur la dégradation de la cornéodesmosine extraite de stratum corneum humain.

### a) Principe

La cornéodesmosine est un marqueur de la desquamation car elle intervient dans la cohésion cornéocytaire au niveau des cornéodesmosomes. Plus la protéine est dégradée, plus le détachement des cornéocytes est significatif. La dégradation de la cornéodesmosine est donc une étape clé de la desquamation.

Le test utilisé dans cette étude consiste donc à suivre cette dégradation en présence de la substance à tester.

### b) Préparation de l'échantillon

Des poudres acétoniques sont préparées à partir de stripping vernis (Méhul B, Bernard D, Simonetti L, Bernard MA, Schmidt R : Identification and cloning of a new calmodulin-like protein from human epidermis. J Biol Chem 275 : 12841-12347, 2000) prélevés sur des bas de jambes de personnes volontaires à peau sèche. Des fractions aliquotes de 2 mg de poudre de *stratum corneum* sont introduites séparément dans des tubes Eppendorfs, puis immergées dans les solutions à tester à raison de 100 µl/mg. Les solutions sont préparées en tampon acétate pH 5. Un témoin sans protéase est préparé en parallèle afin d'évaluer la dégradation naturelle de la cornéodesmosine. Pour chaque test, on prépare trois échantillons. Les échantillons, essais et témoin sont incubés à 30°C sous agitation pendant 24 heures.

### c) Extraction, séparation et détection des protéines.

Les protéines sont extraites avec du tampon Laemmli complet. Elles sont dosées par la méthode de Bradford (kit Bio-Rad^{®}). La concentration de chaque échantillon est ajustée pour permettre la comparaison des échantillons. Les polypeptides sont séparés par électrophorèse SDS-PAGE sur gel d'acrylamide 15 %, puis transférés sur membrane de PVDF. Une immuno-détection par une solution d'anticorps anti-cornéodesmosine utilisée au 1/12500 et d'anticorps secondaires couplés à la peroxydase permet de révéler la cornéodesmosine. Les bandes détectées par chimio-luminescence sont quantifiées à l'aide du logiciel Quantity One ^{®} de la société Biorad. Les membranes sont ensuite colorées à l'amido-black, puis scannées. En outre, les kératines qui sont des protéines majoritaires des extraits cornéocytaires sont quantifiées afin de vérifier l'ajustement à 0,6 mg/ml de tous les échantillons.

Un témoin positif de dégradation (+) est préparé en utilisant 5 mM d'EDTA. Un essai est réalisé en présence de 60 µg de SASPase activée. Un témoin négatif représente la dégradation naturelle de la cornéodesmosine dans les conditions opératoires du test.

### d) Résultats

**TABLEAU II**

| | *Cornéodesmosine normalisée* | | *Cornéodesmosine résiduelle %* |
|---|---|---|---|
| | T0 | T24h | T24h/T0*100 |
| SASPase activée | 33107 | 24918 | 75 |
| Témoin + | 31657 | 26685 | 84 |
| Témoin - | 32701 | 30546 | 93 |

On constate une diminution significative du pourcentage de cornéodesmosine résiduelle lorsque les poudres acétoniques de stratum corneum sont mises en contact avec la SASPase activée (SEQ ID NO : 6) par rapport aux témoins.

### EXEMPLE XII - Recherche de substrat pour la mise en évidence de l'activité SASPase :

On utilise le substrat peptidique considéré (SEQ ID NO : 31, SEQ ID NO :32, SEQ ID NO : 33, SEQ ID NO : 34 ou SEQ ID NO : 35) quenché à l'aide du système fluorescent Abz/(NO2)Tyr à raison de 100µM dans DMSO puis on ajoute 10µL de cette solution mère de substrat pour 100µl de tampon acétate 0,1M pH 5,00.

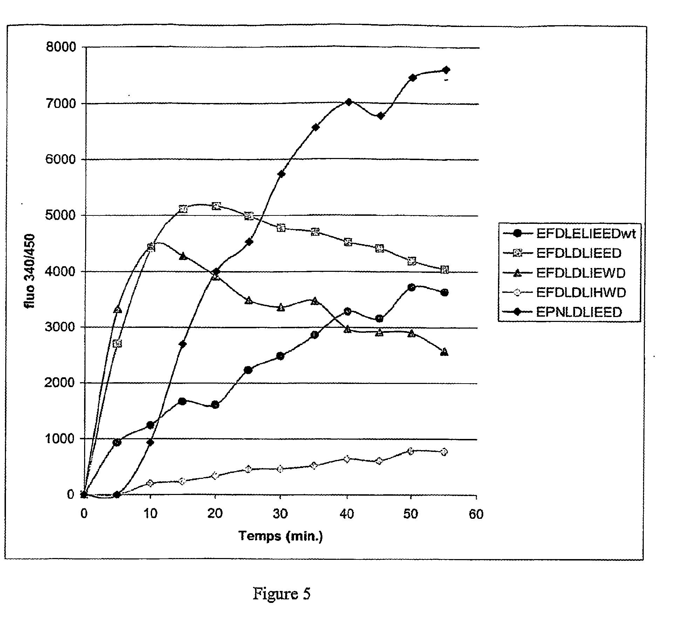

On ajoute 10 µg de GST-C27 (protéine de fusion à 1 mg/ml dans du PBS). Incubation 37°C. La lecture de fluorescence est réalisée à 340/450nm sur lecteur de plaques de type Biolumin à différents temps. Les résultats sont présentés en figure 5.

On note que le peptide naturel (wt) est bien hydrolysé par la SASPase mais des substitutions d'acides aminés dans la séquence de ce peptide peuvent générer des substrats pour lesquels la SASPase possède plus d'affinité.

Une recherche (base MEROPS) sur les différentes protéases capables de couper les liaisons peptidiques hydrolysées ou hydrolysables par la SASPase (combinaisons des différents acides aminés trouvés en position P1 avec les acides aminés trouvés en position P2 des séquences hydrolysées par la SASPase ) font apparaître, en parallèle avec des protéases de type rétroviral, des protéases qui ont été décrites comme importantes pour peau: la SCCE (desquamation, conversion de la proIL1B) les MMP1, 2 et 9 (cicatrisation...), La mu Calpaïn (maturation de l'enveloppe cornée, processing de la filaggrine), la thrombine (activation de « Protease Activated Receptors »), les convertases 1,2,4,5 et 7 (processing de la filaggrine, régulation de la différentiation...).

Ces constatations renforcent l'idée d'un rôle important de la SASPase dans la physiologie cutanée.

## Revendications

1. Composition cosmétique ou pharmaceutique comprenant dans un milieu physiologiquement acceptable, au moins un polypeptide naturel ou synthétique purifié dont la séquence peptidique est représentée par une séquence choisie parmi SEQ ID NO : 6, SEQ ID NO : 16, SEQ ID NO : 25 et SEQ ID NO : 27 et leurs homologues possédant au moins 85 % d'homologie de séquence avec lesdites séquences et la même activité biologique.

2. Composition selon la revendication 1, **caractérisée en ce que** ledit polypeptide se présente sous une forme multimère et de préférence dimère.

3. Composition selon la revendication 1 ou 2, **caractérisée en ce que** ledit polypeptide a subi une ou plusieurs modifications post-traductionnelles.

4. Composition selon l'une quelconque des revendications 1 à 3, **caractérisée en ce que** ledit polypeptide se présente sous la forme d'un polypeptide de séquence SEQ ID NO : 6, SEQ ID NO : 16, SEQ ID NO : 25 ou SEQ ID NO : 27 fusionné avec un agent de ciblage hydrophile ou hydrophobe ou un précurseur de bioconversion.

5. Composition cosmétique ou pharmaceutique comprenant dans un milieu physiologiquement acceptable, au moins un polypeptide naturel ou synthétique purifié dont la séquence peptidique est représentée par une séquence choisie parmi SEQ ID NO : 6, SEQ ID NO : 16, SEQ ID NO : 25 et SEQ ID NO : 27 et leurs homologues possédant au moins 85 % d'homologie de séquence avec lesdites séquences et la même activité biologique en association avec au moins un autre agent actif destiné à la prévention et/ou au traitement des affections cutanées.

6. Procédé de traitement cosmétique destiné à lutter contre les troubles cutanés liés à un dysfonctionnement de la prolifération et/ou de la différenciation cellulaire, **caractérisé en ce que** l'on applique sur la peau, les muqueuses et/ou les fibres kératiniques une composition cosmétique comprenant au moins un polypeptide dont la séquence peptidique est une séquence choisie parmi SEQ ID NO : 6, SEQ ID NO : 16, SEQ ID NO : 25 et SEQ ID NO : 27 et leurs homologues possédant au moins 85 % d'homologie de séquence avec lesdites séquences et la même activité biologique.

7. Procédé de traitement cosmétique selon la revendication 6, **caractérisé en ce qu'**il vise à traiter les peaux sèches, l'hyperkératose, la parakératose, les troubles de la sébogénèse et/ou les signes de vieillissement cutané.

8. Utilisation d'un polypeptide dont la séquence peptidique est une séquence choisie parmi SEQ ID NO : 6, SEQ ID NO : 16, SEQ ID NO : 25 et SEQ ID NO : 27 et leurs homologues possédant au moins 85 % d'homologie de séquence avec lesdites séquences et la même activité biologique pour la préparation d'une composition pharmaceutique destinée au traitement des infections dermatologiques.

9. Utilisation selon la revendication 8, **caractérisée en ce que** la composition pharmaceutique est destinée à traiter l'ichtyose, le psoriasis, l'eczéma, la rosacée, les lichens, les prurits, ou toutes pathologies impliquant une hyperkératose, une parakératose ou ayant une composante inflammatoire.

10. Utilisation d'un polypeptide dont la séquence peptidique est représentée par au moins une séquence choisie parmi SEQ ID NO : 31, SEQ ID NO : 32, SEQ ID NO : 33, SEQ ID NO : 34 et SEQ ID NO : 35, pour préparer une composition destinée à moduler l'activité de la protéase à acide aspartique de séquence SEQ ID NO: 5.

11. Composition cosmétique ou pharmaceutique, **caractérisée en ce qu'**elle comprend dans un milieu physiologiquement acceptable, au moins une séquence nucléotidique codant un polypeptide tel que défini en revendications 1 à 4, ou une séquence sens, antisens ou antisens interférentiel correspondant à ladite séquence nucléotidique.

12. Composition selon la revendication 11, **caractérisée en ce que** ladite séquence nucléotidique est constituée de la séquence nucléotidique codante SEQ ID NO : 20, SEQ ID NO : 24, SEQ ID NO : 26 ou SEQ ID NO : 28.

13. Polypeptide isolé et purifié appartenant à la famille des protéases à acide aspartique, **caractérisé en ce qu'**il possède une séquence peptidique représentée par SEQ ID NO : 6, SEQ ID NO : 16, SEQ ID NO : 25 ou SEQ ID NO : 27.

14. Polypeptide selon la revendication 13, **caractérisé en ce qu'**il possède une masse moléculaire apparente comprise entre 5 et 30 kD, plus particulièrement entre 9 et 15 kD et notamment entre 11 et 14 kD.

15. Polypeptide selon la revendication 13 ou 14, **caractérisé en ce qu'**il se présente sous une forme multimère et de préférence dimère.

16. Polypeptide selon l'une quelconque des revendications 13 à 15, **caractérisé en ce qu'**il possède un point isoélectrique théorique compris entre 3 et 9.

17. Polypeptide selon l'une quelconque des revendications 13 à 16, **caractérisé en ce qu'**il est d'origine naturelle et purifié à partir de tissus de mammifères.

18. Polypeptide selon l'une quelconque des revendications 13 à 17, **caractérisé en ce qu'**il est purifié à partir de peau humaine et plus particulièrement à partir d'épiderme humain.

19. Polypeptide selon l'une quelconque des revendications 13 à 18, **caractérisé en ce qu'**il a subi une ou plusieurs modifications post-traductionnelles.

20. Polypeptide selon la revendication 13 ou l'une des revendications 15 à 19, **caractérisé en ce qu'**il se présente sous la forme d'un polypeptide de séquence SEQ ID NO : 6, SEQ ID NO : 16, SEQ ID NO : 25 ou SEQ ID NO : 27 fusionné avec un agent de ciblage hydrophile ou hydrophobe ou un précurseur de bioconversion.

21. Fragment d'acide désoxyribonucléique isolé et purifié codant un polypeptide tel que défini dans l'une des revendications 14 à 20.

22. Fragment d'acide désoxyribonucléique isolé et purifié constitué de la séquence nucléotidique codante SEQ ID NO : 20, SEQ ID NO : 24, SEQ ID NO : 26 ou SEQ ID NO : 28.

23. Vecteur d'expression recombinant contenant tout ou partie de la séquence nucléotidique codante SEQ ID NO : 20, SEQ ID NO: 24, SEQ ID NO: 26 ou SEQ ID NO : 28.

24. Utilisation d'au moins une séquence d'acide désoxyribonucléique telle que définie en revendications 21 ou 22 pour préparer une séquence d'acide ribonucléique sens, antisens ou antisens interférentiel.

25. Acide ribonucléique, sens, antisens ou antisens interférentiel correspondant au moins à la séquence nucléotidique codante SEQ ID NO : 20, SEQ ID NO : 24, SEQ ID NO : 26 ou SEQ ID NO : 28.

26. Utilisation d'au moins une séquence sens ou antisens telle que définie en revendication 43 à des fins de clonage ou d'identification d'un polypeptide de séquence SEQ ID NO : 6, SEQ ID NO : 16, SEQ ID NO : 25 ou SEQ ID NO : 27 ou de leurs homologues possédant au moins 85 % d'homologie de séquence avec lesdites séquences et la même activité biologique.

27. Utilisation d'au moins une séquence sens ou antisens telle que définie en revendication 43 pour la préparation d'une composition pour une méthode de diagnostic in *vitro* dans laquelle ladite séquence est mise en oeuvre pour la détection d'une synthèse de séquences nucléiques codant pour un polypeptide de séquence SEQ ID NO : 6, SEQ ID NO : 16, SEQ ID NO : 25 ou SEQ ID NO : 27 ou de leurs homologues possédant au moins 85 % d'homologie de séquence avec lesdites séquences et la même activité biologique.

## Claims

1. A cosmetic or pharmaceutical composition comprising, in a physiologically acceptable medium, at least one purified, natural or synthetic polypeptide, the peptide sequence of which is represented by one sequence chosen from, SEQ ID NO : 6, SEQ ID NO : 16, SEQ ID NO : 25 and SEQ ID NO : 27, and homologs having at least 85% sequence homology with saids sequences and the same biological activity.

2. The composition as claimed in claim 1, **characterized in that** said polypeptide is in a multimeric form, and preferably a dimeric form.

3. The composition as claimed in claim 1 or 2, **characterized in that** said polypeptide has undergone one or more post-translational modifications.

4. The composition as claimed in any one of claims 1 to 3, **characterized in that** said polypeptide is in the form of a polypeptide of sequence SEQ ID NO : 6, SEQ ID NO : 16, SEQ ID NO : 25 or SEQ ID NO : 27, fused with another polypeptide, a hydrophilic or hydrophobic targeting agent or a bioconversion precursor.

5. A cosmetic or pharmaceutical composition **characterized in that** it comprises, in a physiologically acceptable medium, at least one purified natural or synthetic polypeptide, the peptide sequence of which is represented by one sequence chosen from SEQ ID NO : 6, SEQ ID NO : 16, SEQ ID NO : 25 or SEQ ID NO : 27, and their homologs having at least 85% sequence homology with saids sequences and the same biological activity, in association with at least one additional active agent for the prevention and/or the treatment of cutaneous diseases.

6. A method of cosmetic treatment intended to combat skin conditions related to a dysfunction of cell proliferation and/or differentiation, **characterized in that** a cosmetic composition comprising at least one polypeptide, the peptide sequence of which is chosen from SEQ ID NO : 6, SEQ ID NO : 16, SEQ ID NO : 25 and SEQ ID NO : 27, and their homologs having at least 85% sequence homology with saids sequences and the same biological activity, is applied to the skin, the mucous membranes and/or the keratin fibers.

7. The method as claimed in claim 6, **characterized in that** it aims to treat dry skin, hyperkeratosis, parakeratosis, sebogenesis conditions, neoplasias and/or signs of skin aging.

8. Use of a polypeptide, the peptide sequence of which is chosen from SEQ ID NO : 6, SEQ ID NO : 16, SEQ ID NO : 25 and SEQ ID NO : 27, and their homologs having at least 85% sequence homology with saids sequences and the same biological activity for preparing a pharmaceutical composition intended for the treatment of dermatological infections.

9. The use as claimed in claim 8, **characterized in that** the pharmaceutical composition is intended to treat ichtyosis, psoriasis, eczema, rosacea, lichens, pruritus, or any pathologies involving hyperkeratosis or parakeratosis or having an inflammatory component.

10. Use of a polypeptide, the peptide sequence of which is represented by at least one sequence chosen from SEQ ID NO : 31, SEQ ID NO : 32, SEQ ID NO : 33, SEQ ID NO : 34 and SEQ ID NO : 35, for preparing a composition intended to modulate the activity of the aspartic acid protease of sequence SEQ ID NO : 5.

11. A cosmetic or pharmaceutical composition, **characterized in that** it comprises, in a physiologically acceptable medium, at least one nucleotide sequence encoding a polypeptide as defined in claims 1 to 4, or a sense, antisense or interfering antisense sequence corresponding to said nucleotide sequence.

12. The composition as claimed in claim 11, **characterized in that** said nucleotide sequence consists of the coding nucleotide sequence SEQ ID NO : 20, SEQ ID NO : 24, SEQ ID NO : 26 or SEQ ID NO : 28.

13. An isolated and purified polypeptide belonging to the aspartic acid protease family, **characterized in that** it has a peptide sequence represented by SEQ ID NO : 6, SEQ ID NO : 16, SEQ ID NO : 25 or SEQ ID NO : 27.

14. The polypeptide as claimed in claim 13, **characterized in that** it has an apparent molecular mass of between 5 and 30 kD, more particularly of between 9 and 15 kD, and especially of between 11 and 14 kD.

15. The polypeptide as claimed in claim 13 or 14, **characterized in that** it is a multimeric form, and preferably a dimeric form.

16. The polypeptide as claimed in any one of claims 13 to 15, **characterized in that** it has a theoretical isoelectric point of between 3 and 9.

17. The polypeptide as claimed in any one of claims 13 to 16, **characterized in that** it is of natural origin and is purified from mammalian tissues.

18. The polypeptide as claimed in any one of claims 13 to 17, **characterized in that** it is purified from human skin, and more particularly from human epidermis.

19. The polypeptide as claimed in any one of claims 13 to 18, **characterized in that** it has undergone one or more post-translational modifications.

20. The polypeptide as claimed in claim 13 or one of claims 15 to 19, **characterized in that** it is in the form of a polypeptide of sequence SEQ ID NO : 6, SEQ ID NO : 16, SEQ ID NO : 25 or SEQ ID NO : 27, fused with another polypeptide, a hydrophilic or hydrophobic targeting agent or a bioconversion precursor.

21. An isolated and purified deoxyribonucleic acid fragment encoding a polypeptide as defined in one of claims 13 to 20.

22. An isolated and purified deoxyribonucleic acid fragment consisting of the coding nucleotide sequence SEQ ID NO : 20, SEQ ID NO : 24, SEQ ID NO : 26 or SEQ ID NO : 28.

23. A recombinant expression vector containing all or part of the coding nucleotide sequence SEQ ID NO : 20, SEQ ID NO : 24, SEQ ID NO : 26 or SEQ ID NO : 28.

24. The use of at least one deoxyribonucleic acid sequence as defined in claim 21 or 22, for preparing a sense, antisense or interfering antisense ribonucleic acid sequence.

25. A sense, antisense or interfering antisense ribonucleic acid corresponding at least to the coding nucleotide sequence SEQ ID NO : 20, SEQ ID NO : 24, SEQ ID NO : 26 or SEQ ID NO : 28.

26. Use of at least one sense or antisense sequence as defined in claim 25, for the purposes of cloning or identifying a polypeptide of sequence SEQ ID NO : 6, SEQ ID NO : 16, SEQ ID NO : 25 or SEQ ID NO : 27, or of homologs having at least 85% sequence homology with saids sequences and the same biological activity.

27. Use of at least one sense or antisense sequence as defined in claim 25, for preparing a composition intended for the diagnosis of a polypeptide of sequence, SEQ ID NO : 6, SEQ ID NO : 16, SEQ ID NO : 25 or SEQ ID NO : 27, or of homologs having at least 85% sequence homology with saids sequences and the same biological activity.

## Patentansprüche

1. Kosmetische oder pharmazeutische Zusammensetzung, umfassend, in einem physiologisch verträglichen Medium, mindestens ein natürliches oder synthetisches gereinigtes Polypeptid, dessen Peptidsequenz durch eine Sequenz dargestellt ist, die ausgewählt ist aus SEQ ID NO: 6, SEQ ID NO: 16, SEQ ID NO: 25 und SEQ ID NO: 27 und ihren Homologen, die mindestens 85 % Sequenzhomologie mit den Sequenzen und die gleiche biologische Aktivität besitzen.

2. Zusammensetzung nach Anspruch 1, **dadurch gekennzeichnet, dass** das Polypeptid in einer multimeren und vorzugsweise dimeren Form vorliegt.

3. Zusammensetzung nach Anspruch 1 oder 2, **dadurch gekennzeichnet, dass** das Polypeptid eine oder mehrere posttranslationelle Modifikationen durchlaufen hat.

4. Zusammensetzung nach einem der Ansprüche 1 bis 3, **dadurch gekennzeichnet, dass** das Polypeptid in der Form eines Polypeptids der Sequenz SEQ ID NO: 6, SEQ ID NO: 16, SEQ ID NO: 25 oder SEQ ID NO: 27, fusioniert mit einem hydrophilen oder hydrophoben Targeting-Wirkstoffoder einem Biokonversionsvorläufer, vorliegt.

5. Kosmetische oder pharmazeutische Zusammensetzung, umfassend, in einem physiologisch verträglichen Medium, mindestens ein natürliches oder synthetisches gereinigtes Polypeptid, dessen Peptidsequenz durch eine Sequenz dargestellt ist, die ausgewählt ist aus SEQ ID NO: 6, SEQ ID NO: 16, SEQ ID NO: 25 und SEQ ID NO: 27 und ihren Homologen, die mindestens 85 % Sequenzhomologie mit den Sequenzen und die gleiche biologische Aktivität besitzen, in Kombination mit mindestens einem anderen Wirkstoff, der zur Verhinderung und/oder Behandlung von Hautleiden bestimmt ist.

6. Kosmetisches Behandlungsverfahren, das zur Bekämpfung von Hautbeschwerden bestimmt ist, die mit einer Fehlfunktion der Proliferation und/oder der Zelldifferenzierung zusammenhängen, **dadurch gekennzeichnet, dass** auf die Haut, die Schleimhäute und/oder die Keratinfasern eine kosmetische Zusammensetzung aufgetragen wird, die mindestens ein Polypeptid umfasst, dessen Peptidsequenz eine Sequenz ist, die ausgewählt ist aus SEQ ID NO: 6, SEQ ID NO: 16, SEQ ID NO: 25 und SEQ ID NO: 27 und ihren Homologen, die mindestens 85 % Sequenzhomologie mit den Sequenzen und die gleiche biologische Aktivität besitzen.

7. Kosmetisches Behandlungsverfahren nach Anspruch 6, **dadurch gekennzeichnet, dass** es darauf ausgerichtet ist, trockene Häute, Hyperkeratose, Parakeratose, Störungen der Sebogenese und/oder Anzeichen der Hautalterung zu behandeln.

8. Verwendung eines Polypeptids, dessen Peptidsequenz eine Sequenz ist, die ausgewählt ist aus SEQ ID NO: 6, SEQ ID NO: 16, SEQ ID NO: 25 und SEQ ID NO: 27 und ihren Homologen, die mindestens 85 % Sequenzhomologie mit den Sequenzen und die gleiche biologische Aktivität besitzen, zur Herstellung einer pharmazeutischen Zusammensetzung, die zur Behandlung von dermatologischen Infektionen bestimmt ist.

9. Verwendung nach Anspruch 8, **dadurch gekennzeichnet, dass** die pharmazeutische Zusammensetzung zur Behandlung von Ichtyose, Psoriasis, Ekzem, Rosazea, Flechten, Pruritus, oder allen Pathologien, an denen eine Hyperkeratose, eine Parakerastose beteiligt ist, oder mit einer Entzündungskomponente, bestimmt ist.

10. Verwendung eines Polypeptids, dessen Peptidsequenz durch mindestens eine Sequenz dargestellt ist, ausgewählt aus SEQ ID NO: 31, SEQ ID NO: 32, SEQ ID NO: 33, SEQ ID NO: 34 und SEQ ID NO: 35, zur Herstellung einer Zusammensetzung, die zur Modulierung der Aktivität der Asparaginsäureprotease der Sequenz SEQ ID NO: 5 bestimmt ist.

11. Kosmetische oder pharmazeutische Zusammensetzung, **dadurch gekennzeichnet, dass** sie, in einem physiologisch verträglichen Medium, mindestens eine Nucleotidsequenz, die ein Polypeptid kodiert, wie in den Ansprüchen 1 bis 4 definiert, oder eine sense-, antisense- oder interferentielle antisense-Sequenz, entsprechend der Nucleotidsequenz, umfasst.

12. Zusammensetzung nach Anspruch 11, **dadurch gekennzeichnet, dass** die Nucleotidsequenz aus der Nucleotidsequenz aufgebaut ist, die SEQ ID NO: 20, SEQ ID NO: 24, SEQ ID NO: 26, SEQ ID NO: 34 oder SEQ ID NO: 28 kodiert.

13. Isoliertes und gereinigtes Polypetptid, das der Familie der Asparaginsäureproteasen angehört, **dadurch gekennzeichnet, dass** es eine durch SEQ ID NO: 6, SEQ ID NO: 16, SEQ ID NO: 25 oder SEQ ID NO: 27 dargestellte Peptidsequenz besitzt.

14. Polypeptid nach Anspruch 13, **dadurch gekennzeichnet dass** es eine scheinbare Molekülmasse zwischen 5 und 30 kD, spezieller zwischen 9 und 15 kD, und insbesondere zwischen 11 und 14 kD, Grenzen eingeschlossen, besitzt.

15. Polypeptid nach Anspruch 13 oder 14, **dadurch gekennzeichnet dass** es in einer multimeren und vorzugsweise dimeren Form vorliegt.

16. Polypeptid nach einem der Ansprüche 13 bis 15, **dadurch gekennzeichnet dass** es einen theoretischen isoelektrischen Punkt zwischen 3 und 9, Grenzen eingeschlossen, besitzt.

17. Polypeptid nach einem der Ansprüche 13 bis 16, **dadurch gekennzeichnet dass** es natürlicher Herkunft ist und aus Geweben von Säugern aufgereinigt ist.

18. Polypeptid nach einem der Ansprüche 13 bis 17, **dadurch gekennzeichnet dass** es aus menschlicher Haut und insbesondere aus menschlicher Epidermis aufgereinigt ist.

19. Polypeptid nach einem der Ansprüche 13 bis 18, **dadurch gekennzeichnet dass** es eine oder mehrere posttranslationelle Modifikationen durchlaufen hat.

20. Polypeptid nach Anspruch 13 oder einem der Ansprüche 15 bis 19, **dadurch gekennzeichnet, dass** es in der Form eines Polypeptids der Sequenz SEQ ID NO: 6, SEQ ID NO: 16, SEQ ID NO: 25 oder SEQ ID NO: 27, fusioniert mit einem hydrophilen oder hydrophoben Targeting-Wirkstoff oder einem Biokonversionsvorläufer, vorliegt.

21. Isoliertes und gereinigtes Desoxyribonucleinsäurefragment, das ein Polypeptid, wie in einem der Ansprüche 13 bis 20 definiert, kodiert.

22. Isoliertes und gereinigtes Desoxyribonucleinsäurefragment, das aus der kodierenden Nucleotidsequenz SEQ ID NO: 20, SEQ ID NO: 24, SEQ ID NO: 26 oder SEQ ID NO: 28 besteht.

23. Rekombinanter Expressionsvektor, der alles oder einen Teil der kodierenden Nucleotidsequenz SEQ ID NO: 20, SEQ ID NO: 24, SEQ ID NO: 26 oder SEQ ID NO: 28 enthält.

24. Verwendung von mindestens einer Desoxyribonucleinsäuresequenz, wie in den Ansprüchen 21 oder 22 definiert, zur Herstellung einer sense-, antisense- oder interferentiellen antisense-Ribonucleinsäuresequenz.

25. Sense-, antisense- oder interferentielle antisense-Ribonucleinsäuresequenz, entsprechend mindestens der kodierenden Nucleotidsequenz SEQ ID NO: 20, SEQ ID NO: 24, SEQ ID NO: 26 oder SEQ ID NO: 28.

26. Verwendung von mindestens einer sense- oder antisense-Sequenz, wie in Anspruch 25 definiert, zur Klonierung oder Identifizierung eines Polypeptids der Sequenz SEQ ID NO: 6, SEQ ID NO: 16, SEQ ID NO: 25 oder SEQ ID NO: 27 oder ihren Homologen, die mindestens 85 % Sequenzhomologie mit den Sequenzen und die gleiche biologische Aktivität besitzen.

27. Verwendung von mindestens einer sense- oder antisense-Sequenz, wie in Anspruch 25 definiert, zur Herstellung einer Zusammensetzung für ein diagnostisches *in vitro* Verfahren, wobei die Sequenz zum Nachweis einer Synthese von Nucleinsäuresequenzen eingesetzt wird, die ein Polypeptid der Sequenz SEQ ID NO: 6, SEQ ID NO: 16, SEQ ID NO: 25 oder SEQ ID NO: 27 kodieren, oder ihren Homologen, die mindestens 85 % Sequenzhomologie mit den Sequenzen und die gleiche biologische Aktivität besitzen.


### SEQUENCE LISTING

<110> L'OREAL
<120> Utilisation de protéases aspartiques dans le domaine cosmétique et thérapeutique.
<130> BR75585/CR/PLC/cr
<150> FR 02 08613
   <151> 2002-07-09
<160> 36
<170> PatentIn version 3.1
<210> 1
   <211> 12
   <212> PRT
   <213> Homo sapiens
<400> 1
   Phe Leu Val Asp Ser Gly Ala Gln Val Ser Val Val
   1               5                   10
<210> 2
   <211> 21
   <212> PRT
   <213> Homo Sapiens
<400> 2
   Ala Gln Phe Leu Val Ala Asn Ala Ser Ala Glu Glu Ala Ile Ile Gly
   1               5                   10                  15
   Thr Asp Val Leu Gln
               20
<210> 3
   <211> 9
   <212> PRT
   <213> Homo Sapiens
<400> 3
   Ile Leu Gly Val Trp Asp Thr Ala Val
   1               5
<210> 4
   <211> 259
   <212> PRT
   <213> Homo Sapiens
<400> 4
   Met Ala Gly Ser Gly Ala Arg Ser Glu Glu Gly Arg Arg Gln His Ala
   1               5                   10                  15
   Phe Val Pro Glu Pro Phe Asp Gly Ala Asn Val Val Pro Asn Leu Trp
               20                  25                  30
   Leu His Ser Phe Glu Val Ile Asn Asp Leu Asn His Trp Asp His Ile
           35                  40                  45
   Thr Lys Leu Arg Phe Leu Lys Glu Ser Leu Arg Gly Glu Ala Leu Gly
       50                  55                  60
   Val Tyr Asn Arg Leu Ser Pro Gln Asp Gln Gly Asp Tyr Gly Thr Val
   65                  70                  75                  80
   Lys Glu Ala Leu Leu Lys Ala Phe Gly Val Pro Gly Ala Ala Pro Ser
                   85                  90                  95
   His Leu Pro Lys Glu Ile Val Phe Ala Asn Ser Met Gly Lys Gly Tyr
               100                 105                 110
   Tyr Leu Lys Gly Lys Ile Gly Lys Val Pro Val Arg Phe Leu Val Asp
           115                 120                 125
   Ser Gly Ala Gln Val Ser Val Val His Pro Asn Leu Trp Glu Glu Val
       130                 135                 140
   Thr Asp Gly Asp Leu Asp Thr Leu Gln Pro Phe Glu Asn Val Val Lys
   145                 150                 155                 160
   Val Ala Asn Gly Ala Glu Met Lys Ile Leu Gly Val Trp Asp Thr Ala
                   165                 170                 175
   Val Ser Leu Gly Lys Leu Lys Leu Lys Ala Gln Phe Leu Val Ala Asn
               180                 185                 190
   Ala Ser Ala Glu Glu Ala Ile Ile Gly Thr Asp Val Leu Gln Asp His
           195                 200                 205
   Asn Ala Ile Leu Asp Phe Glu His Arg Thr Cys Thr Leu Lys Gly Lys
       210                 215                 220
   Lys Phe Arg Leu Leu Pro Val Gly Gly Ser Leu Glu Asp Glu Phe Asp
   225                 230                 235                 240
   Leu Glu Leu Ile Glu Glu Asp Pro Ser Ser Glu Glu Gly Arg Gln Glu
                   245                 250                 255
   Leu Ser His
<210> 5
   <211> 343
   <212> PRT
   <213> Homo Sapiens
<400> 5
   Met Gly Ser Pro Gly Ala Ser Leu Gly Ile Lys Lys Ala Leu Gln Ser
   1               5                   10                  15
   Glu Gln Ala Thr Ala Leu Pro Ala Ser Ala Pro Ala Val Ser Gln Pro
               20                  25                  30
   Thr Ala Pro Ala Pro Ser Cys Leu Pro Lys Ala Gly Gln Val Ile Pro
           35                  40                  45
   Thr Leu Leu Arg Glu Ala Pro Phe Ser Ser Val Ile Ala Pro Thr Leu
   50 55 60
   Leu Cys Gly Phe Leu Phe Leu Ala Trp Val Ala Ala Glu Val Pro Glu
   65 70 75 80
   Glu Ser Ser Arg Met Ala Gly Ser Gly Ala Arg Ser Glu Glu Gly Arg
   85 90 95
   Arg Gln His Ala Phe Val Pro Glu Pro Phe Asp Gly Ala Asn Val Val
   100 105 110
   Pro Asn Leu Trp Leu His Ser Phe Glu Val Ile Asn Asp Leu Asn His
   115 120 125
   Trp Asp His Ile Thr Lys Leu Arg Phe Leu Lys Glu Ser Leu Arg Gly
   130 135 140
   Glu Ala Leu Gly Val Tyr Asn Arg Leu Ser Pro Gln Asp Gln Gly Asp
   145 150 155 160
   Tyr Gly Thr Val Lys Glu Ala Leu Leu Lys Ala Phe Gly Val Pro Gly
   165 170 175
   Ala Ala Pro Ser His Leu Pro Lys Glu Ile Val Phe Ala Asn Ser Met
   180 185 190
   Gly Lys Gly Tyr Tyr Leu Lys Gly Lys Ile Gly Lys Val Pro Val Arg
   195 200 205
   Phe Leu Val Asp Ser Gly Ala Gln Val Ser Val Val His Pro Asn Leu
   210 215 220
   Trp Glu Glu Val Thr Asp Gly Asp Leu Asp Thr Leu Gln Pro Phe Glu
   225 230 235 240
   Asn Val Val Lys Val Ala Asn Gly Ala Glu Met Lys Ile Leu Gly Val
   245 250 255
   Trp Asp Thr Ala Val Ser Leu Gly Lys Leu Lys Leu Lys Ala Gln Phe
   260 265 270
   Leu Val Ala Asn Ala Ser Ala Glu Glu Ala Ile Ile Gly Thr Asp Val
   275 280 285
   Leu Gln Asp His Asn Ala Ile Leu Asp Phe Glu His Arg Thr Cys Thr
   290 295 300
   Leu Lys Gly Lys Lys Phe Arg Leu Leu Pro Val Gly Gly Ser Leu Glu
   305 310 315 320
   Asp Glu Phe Asp Leu Glu Leu Ile Glu Glu Asp Pro Ser Ser Glu Glu
   325 330 335
   Gly Arg Gln Glu Leu Ser His
   340
<210> 6
   <211> 138
   <212> PRT
   <213> Homo Sapiens
<400> 6
   Ala Asn Ser Met Gly Lys Gly Tyr Tyr Leu Lys Gly Lys Ile Gly Lys
   1 5 10 15
   Val Pro Val Arg Phe Leu Val Asp Ser Gly Ala Gln Val Ser Val Val
   20 25 30
   His Pro Asn Leu Trp Glu Glu Val Thr Asp Gly Asp Leu Asp Thr Leu
   35 40 45
   Gln Pro Phe Glu Asn Val Val Lys Val Ala Asn Gly Ala Glu Met Lys
   50 55 60
   Ile Leu Gly Val Trp Asp Thr Ala Val Ser Leu Gly Lys Leu Lys Leu
   65 70 75 80
   Lys Ala Gln Phe Leu Val Ala Asn Ala Ser Ala Glu Glu Ala Ile Ile
   85 90 95
   Gly Thr Asp Val Leu Gln Asp His Asn Ala Ile Leu Asp Phe Glu His
   100 105 110
   Arg Thr Cys Thr Leu Lys Gly Lys Lys Phe Arg Leu Leu Pro Val Gly
   115 120 125
   Gly Ser Leu Glu Asp Glu Phe Asp Leu Glu
   130 135
<210> 7
   <211> 58
   <212> PRT
   <213> Homo Sapiens
<400> 7
   Met Gly Ser Pro Gly Ala Ser Leu Gly Ile Lys Lys Ala Leu Gln Ser
   1 5 10 15
   Glu Gln Ala Thr Ala Leu Pro Ala Ser Ala Pro Ala Val Ser Gln Pro
   20 25 30
   Thr Ala Pro Ala Pro Ser Cys Leu Pro Lys Ala Gly Gln Val Ile Pro
   35 40 45
   Thr Leu Leu Arg Glu Ala Pro Phe Ser Ser
   50 55
<210> 8
   <211> 18
   <212> PRT
   <213> Homo sapiens
<400> 8
   Val Ile Ala Pro Thr Leu Leu Cys Gly Phe Leu Phe Leu Ala Trp Val
   1 5 10 15
   Ala Ala
<210> 9
   <211> 17
   <212> PRT
   <213> Homo Sapiens
<400> 9
   Leu Ile Glu Glu Asp Pro Ser Ser Glu Glu Gly Arg Gln Glu Leu Ser
   1 5 10 15
   His
<210> 10
   <211> 25
   <212> DNA
   <213> Artificial Sequence
<220>
   <223> Amorce SC140 d'amplification par PCR de fragments d'ADNc codant pour la SASPase complète.
<400> 10
   taggatccat ggggagccca ggggc 25
<210> 11
   <211> 30
   <212> DNA
   <213> Artificial Sequence
<220>
   <223> Amorce SC131 d'amplification par PCR d'ADNc codant pour la SASPase complète ou pour la forme
   tronquée SASPase Delta 1-84.
<400> 11
   ttgaattctc agtgggatag ctcctgccgc 30
<210> 12
   <211> 33
   <212> DNA
   <213> Artificial Sequence
<220>
   <223> Amorce SC130 d'amplification par PCR d'ADNc codant pour la SASPase dite Delta 1-84.
<400> 12
   gataggatcc atggccggga gcggagccag gag 33
<210> 13
   <211> 20
   <212> DNA
   <213> Artificial Sequence
<220>
   <223> Amorce SC134 d'amplification par RT-PCR.
<400> 13
   ggccctgggt gtctacaata 20
<210> 14
   <211> 20
   <212> DNA
   <213> Artificial Sequence
<220>
   <223> Amorce SC135 d'amplification par RT-PCR.
<400> 14
   ttggccacct ttaccacatt 20
<210> 15
   <211> 25
   <212> PRT
   <213> Homo Sapiens
<400> 15
   Phe Val Asn Gln His Leu Cys Gly Ser His Leu Val Glu Ala Leu Tyr
   1               5                   10                  15
   Leu Val Cys Gly Glu Arg Gly Phe Phe
               20                  25
<210> 16
   <211> 136
   <212> PRT
   <213> Homo Sapiens
<400> 16
<210> 17
   <211> 36
   <212> DNA
   <213> Homo Sapiens
<220>
   <221> CDS
   <222> (1) .. (36)
   <223>
<400> 17
   ttc ctg gtg gac tct ggg gcc cag gtc tct gtg gtc 36
   Phe Leu Val Asp Ser Gly Ala Gln Val Ser Val Val
   1 5 10
<210> 18
   <211> 777
   <212> DNA
   <213> Homo Sapiens
<220>
   <221> CDs
   <222> (1) .. (777)
   <223>
<400> 18
   atg gcc ggg agc gga gcc agg agt gag gaa ggc cgc cgg cag cat gcc 48
   Met Ala Gly Ser Gly Ala Arg Ser Glu Glu Gly Arg Arg Gln His Ala
   1 5 10 15
   ttc gtc ccg gaa cct ttt gat ggg gcc aat gtc gtc cca aac ctc tgg 96
   Phe Val Pro Glu Pro Phe Asp Gly Ala Asn Val Val Pro Asn Leu Trp
   20 25 30
   ctg cac agc ttt gaa gtc atc aat gac ctc aac cat tgg gac cat atc 144
   Leu His Ser Phe Glu Val Ile Asn Asp Leu Asn His Trp Asp His Ile
   35 40 45
   acc aag cta agg ttc ctg aaa gag tcc ctc aga gga gag gcc ctg ggt 192
   Thr Lys Leu Arg Phe Leu Lys Glu Ser Leu Arg Gly Glu Ala Leu Gly
   50 55 60
   gtc tac aat agg ctc agt ccc cag gac cag gga gac tat ggg act gtg 240
   Val Tyr Asn Arg Leu Ser Pro Gln Asp Gln Gly Asp Tyr Gly Thr Val
   65 70 75 80
   aaa gag gcc ctc ctg aag gcc ttt ggg gtc cct ggg gct gcc ccc agc 288
   Lys Glu Ala Leu Leu Lys Ala Phe Gly Val Pro Gly Ala Ala Pro Ser
   85 90 95
   cac ctg ccc aaa gag atc gtc ttt gcc aac agc atg ggt aag ggc tac 336
   His Leu Pro Lys Glu Ile Val Phe Ala Asn Ser Met Gly Lys Gly Tyr
   100 105 110
   tat ctc aag ggg aag att ggc aaa gtg ccc gtg agg ttc ctg gtg gac 384
   Tyr Leu Lys Gly Lys Ile Gly Lys Val Pro Val Arg Phe Leu Val Asp
   115 120 125
   tct ggg gcc cag gtc tct gtg gtc cac cca aac ttg tgg gag gag gtc 432
   Ser Gly Ala Gln Val Ser Val Val His Pro Asn Leu Trp Glu Glu Val
   130 135 140
   act gat ggc gat ctg gac acc ctg cag ccc ttt gag aat gtg gta aag 480
   Thr Asp Gly Asp Leu Asp Thr Leu Gln Pro Phe Glu Asn Val Val Lys
   145 150 155 160
   gtg gcc aat ggt gct gaa atg aag atc ctg ggt gtc tgg gat aca gcg 528
   Val Ala Asn Gly Ala Glu Met Lys Ile Leu Gly Val Trp Asp Thr Ala
   165 170 175
   gtg tcc cta ggc aag ctg aag ctg aag gca cag ttc cta gtg gcc aat 576
   Val Ser Leu Gly Lys Leu Lys Leu Lys Ala Gln Phe Leu Val Ala Asn
   180 185 190
   gcg agt gcc gag gaa gcc atc att ggc act gat gtg ctc cag gac cac 624
   Ala Ser Ala Glu Glu Ala Ile Ile Gly Thr Asp Val Leu Gln Asp His
   195 200 205
   aat gct atc ctg gac ttt gag cac cgc aca tgc acc ctg aaa ggg aag 672
   Asn Ala Ile Leu Asp Phe Glu His Arg Thr Cys Thr Leu Lys Gly Lys
   210 215 220
   aag ttt cgc ctt ctg cct gtg gga ggg tcc ctg gaa gat gag ttt gac 720
   Lys Phe Arg Leu Leu Pro Val Gly Gly Ser Leu Glu Asp Glu Phe Asp
   225 230 235 240
   ctg gag ctc ata gag gag gac ccc tcc tca gaa gaa ggg cgg cag gag 768
   Leu Glu Leu Ile Glu Glu Asp Pro Ser Ser Glu Glu Gly Arg Gln Glu
   245 250 255
   cta tcc cac 777
   Leu Ser His
<210> 19
   <211> 1029
   <212> DNA
   <213> Homo Sapiens
<220>
   <221> CDS
   <222> (1) .. (1029)
   <223>
<400> 19
   atg ggg agc cca ggg gcc agc cta ggc atc aaa aag gct ctg cag agt       48
   Met Gly Ser Pro Gly Ala Ser Leu Gly Ile Lys Lys Ala Leu Gln Ser
   1               5                   10                  15
   gaa cag gcc aca gca ctg cct gcc tct gcc cca gca gtc agc cag ccg       96
   Glu Gln Ala Thr Ala Leu Pro Ala Ser Ala Pro Ala Val Ser Gln Pro
               20                  25                  30
   acc gcg cct gct ccc tcc tgc ttg ccc aag gcc gga caa gtc atc ccc      144
   Thr Ala Pro Ala Pro Ser Cys Leu Pro Lys Ala Gly Gln Val Ile Pro
           35                  40                  45
   act ctg ctt cga gag gcc ccg ttt tcc agc gtg att gcg ccg aca ctg      192
   Thr Leu Leu Arg Glu Ala Pro Phe Ser Ser Val Ile Ala Pro Thr Leu
       50                  55                  60
   ctc tgt ggg ttt ctc ttc ttg gcg tgg gtt gct gct gag gtt cca gag      240
   Leu Cys Gly Phe Leu Phe Leu Ala Trp Val Ala Ala Glu Val Pro Glu
   65                  70                  75                  80
   gag agc agc agg atg gcc ggg agc gga gcc agg agt gag gaa ggc cgc      288
   Glu Ser Ser Arg Met Ala Gly Ser Gly Ala Arg Ser Glu Glu Gly Arg
                   85                  90                  95
   cgg cag cat gcc ttc gtc ccg gaa cct ttt gat ggg gcc aat gtc gtc      336
   Arg Gln His Ala Phe Val Pro Glu Pro Phe Asp Gly Ala Asn Val Val
               100                 105                 110
   cca aac ctc tgg ctg cac agc ttt gaa gtc atc aat gac ctc aac cat      384
   Pro Asn Leu Trp Leu His Ser Phe Glu Val Ile Asn Asp Leu Asn His
           115                 120                 125
   tgg gac cat atc acc aag cta agg ttc ctg aaa gag tcc ctc aga gga      432
   Trp Asp His Ile Thr Lys Leu Arg Phe Leu Lys Glu Ser Leu Arg Gly
       130                 135                 140
   gag gcc ctg ggt gtc tac aat agg ctc agt ccc cag gac cag gga gac      480
   Glu Ala Leu Gly Val Tyr Asn Arg Leu Ser Pro Gln Asp Gln Gly Asp
   145                 150                 155                 160
   tat ggg act gtg aaa gag gcc ctc ctg aag gcc ttt ggg gtc cct ggg      528
   Tyr Gly Thr Val Lys Glu Ala Leu Leu Lys Ala Phe Gly Val Pro Gly
                   165                 170                 175
   gct gcc ccc agc cac ctg ccc aaa gag atc gtc ttt gcc aac agc atg      576
   Ala Ala Pro Ser His Leu Pro Lys Glu Ile Val Phe Ala Asn Ser Met
               180                 185                 190
   ggt aag ggc tac tat ctc aag ggg aag att ggc aaa gtg ccc gtg agg      624
   Gly Lys Gly Tyr Tyr Leu Lys Gly Lys Ile Gly Lys Val Pro Val Arg
           195                 200                 205
   ttc ctg gtg gac tct ggg gcc cag gtc tct gtg gtc cac cca aac ttg      672
   Phe Leu Val Asp Ser Gly Ala Gln Val Ser Val Val His Pro Asn Leu
       210                 215                 220
   tgg gag gag gtc act gat ggc gat ctg gac acc ctg cag ccc ttt gag      720
   Trp Glu Glu Val Thr Asp Gly Asp Leu Asp Thr Leu Gln Pro Phe Glu
   225                 230                 235                 240
   aat gtg gta aag gtg gcc aat ggt gct gaa atg aag atc ctg ggt gtc      768
   Asn Val Val Lys Val Ala Asn Gly Ala Glu Met Lys Ile Leu Gly Val
                   245                 250                 255
   tgg gat aca gcg gtg tcc cta ggc aag ctg aag ctg aag gca cag ttc      816
   Trp Asp Thr Ala Val Ser Leu Gly Lys Leu Lys Leu Lys Ala Gln Phe
               260                 265                 270
   cta gtg gcc aat gcg agt gcc gag gaa gcc atc att ggc act gat gtg      864
   Leu Val Ala Asn Ala Ser Ala Glu Glu Ala Ile Ile Gly Thr Asp Val
           275                 280                 285
   ctc cag gac cac aat gct atc ctg gac ttt gag cac cgc aca tgc acc      912
   Leu Gln Asp His Asn Ala Ile Leu Asp Phe Glu His Arg Thr Cys Thr
       290                 295                 300
   ctg aaa ggg aag aag ttt cgc ctt ctg cct gtg gga ggg tcc ctg gaa      960
   Leu Lys Gly Lys Lys Phe Arg Leu Leu Pro Val Gly Gly Ser Leu Glu
   305                 310                 315                 320
   gat gag ttt gac ctg gag ctc ata gag gag gac ccc tcc tca gaa gaa     1008
   Asp Glu Phe Asp Leu Glu Leu Ile Glu Glu Asp Pro Ser Ser Glu Glu
                   325                 330                 335
   ggg cgg cag gag cta tcc cac                                         1029
   Gly Arg Gln Glu Leu Ser His
               340
<210> 20
   <211> 414
   <212> DNA
   <213> Homo Sapiens
<220>
   <221> CDS
   <222> (1)..(414)
   <223>
<400> 20
   gcc aac agc atg ggt aag ggc tac tat ctc aag ggg aag att ggc aaa      48
   Ala Asn Ser Met Gly Lys Gly Tyr Tyr Leu Lys Gly Lys Ile Gly Lys
   1               5                   10                  15
   gtg ccc gtg agg ttc ctg gtg gac tct ggg gcc cag gtc tct gtg gtc      96
   Val Pro Val Arg Phe Leu Val Asp Ser Gly Ala Gln Val Ser Val Val
               20                  25                  30
   cac cca aac ttg tgg gag gag gtc act gat ggc gat ctg gac acc ctg     144
   His Pro Asn Leu Trp Glu Glu Val Thr Asp Gly Asp Leu Asp Thr Leu
           35                  40                  45
   cag ccc ttt gag aat gtg gta aag gtg gcc aat ggt gct gaa atg aag     192
   Gln Pro Phe Glu Asn Val Val Lys Val Ala Asn Gly Ala Glu Met Lys
       50                  55                  60
   atc ctg ggt gtc tgg gat aca gcg gtg tcc cta ggc aag ctg aag ctg     240
   Ile Leu Gly Val Trp Asp Thr Ala Val Ser Leu Gly Lys Leu Lys Leu
   65                  70                  75                  80
   aag gca cag ttc cta gtg gcc aat gcg agt gcc gag gaa gcc atc att     288
   Lys Ala Gln Phe Leu Val Ala Asn Ala Ser Ala Glu Glu Ala Ile Ile
                   85                  90                  95
   ggc act gat gtg ctc cag gac cac aat gct atc ctg gac ttt gag cac     336
   Gly Thr Asp Val Leu Gln Asp His Asn Ala Ile Leu Asp Phe Glu His
               100                 105                 110
   cgc aca tgc acc ctg aaa ggg aag aag ttt cgc ctt ctg cct gtg gga     384
   Arg Thr Cys Thr Leu Lys Gly Lys Lys Phe Arg Leu Leu Pro Val Gly
           115                 120                 125
   ggg tcc ctg gaa gat gag ttt gac ctg gag                             414
   Gly Ser Leu Glu Asp Glu Phe Asp Leu Glu
       130                 135
<210> 21
   <211> 174
   <212> DNA
   <213> Homo Sapiens
<220>
   <221> CDS
   <222> (1) .. (174)
   <223>
<400> 21
   atg ggg agc cca ggg gcc agc cta ggc atc aaa aag gct ctg cag agt      48
   Met Gly Ser Pro Gly Ala Ser Leu Gly Ile Lys Lys Ala Leu Gln Ser
   1               5                   10                  15
   gaa cag gcc aca gca ctg cct gcc tct gcc cca gca gtc agc cag ccg      96
   Glu Gln Ala Thr Ala Leu Pro Ala Ser Ala Pro Ala Val Ser Gln Pro
               20                  25                  30
   acc gcg cct gct ccc tcc tgc ttg ccc aag gcc gga caa gtc atc ccc     144
   Thr Ala Pro Ala Pro Ser Cys Leu Pro Lys Ala Gly Gln Val Ile Pro
           35                  40                  45
   act ctg ctt cga gag gcc ccg ttt tcc agc                             174
   Thr Leu Leu Arg Glu Ala Pro Phe Ser Ser
       50                  55
<210> 22
   <211> 54
   <212> DNA
   <213> Homo Sapiens
<220>
   <221> CDS
   <222> (1) .. (54)
   <223>
<400> 22
   gtg att gcg ccg aca ctg ctc tgt ggg ttt ctc ttc ttg gcg tgg gtt      48
   Val Ile Ala Pro Thr Leu Leu Cys Gly Phe Leu Phe Leu Ala Trp Val
   1               5                   10                  15
   gct gct                                                              54
   Ala Ala
<210> 23
   <211> 51
   <212> DNA
   <213> Homo Sapiens
<220>
   <221> CDS
   <222> (1) .. (51)
   <223>
<400> 23
   ctc ata gag gag gac ccc tcc tca gaa gaa ggg cgg cag gag cta tcc      48
   Leu Ile Glu Glu Asp Pro Ser Ser Glu Glu Gly Arg Gln Glu Leu Ser
   1               5                   10                  15
   cac                                                                  51
   His
<210> 24
   <211> 408
   <212> DNA
   <213> Homo Sapiens
<220>
   <221> CDS
   <222> (1)..(408)
   <223>
<400> 24
   agc atg ggt aag ggc tac tat ctc aag ggg aag att ggc aaa gtg ccc        48
   Ser Met Gly Lys Gly Tyr Tyr Leu Lys Gly Lys Ile Gly Lys Val Pro
   1               5                   10                  15
   gtg agg ttc ctg gtg gac tct ggg gcc cag gtc tct gtg gtc cac cca        96
   Val Arg Phe Leu Val Asp Ser Gly Ala Gln Val Ser Val Val His Pro
               20                  25                  30
   aac ttg tgg gag gag gtc act gat ggc gat ctg gac acc ctg cag ccc       144
   Asn Leu Trp Glu Glu Val Thr Asp Gly Asp Leu Asp Thr Leu Gln Pro
           35                  40                  45
   ttt gag aat gtg gta aag gtg gcc aat ggt gct gaa atg aag atc ctg       192
   Phe Glu Asn Val Val Lys Val Ala Asn Gly Ala Glu Met Lys Ile Leu
       50                  55                  60
   ggt gtc tgg gat aca gcg gtg tcc cta ggc aag ctg aag ctg aag gca       240
   Gly Val Trp Asp Thr Ala Val Ser Leu Gly Lys Leu Lys Leu Lys Ala
   65                  70                  75                  80
   cag ttc cta gtg gcc aat gcg agt gcc gag gaa gcc atc att ggc act       288
   Gln Phe Leu Val Ala Asn Ala Ser Ala Glu Glu Ala Ile Ile Gly Thr
                   85                  90                  95
   gat gtg ctc cag gac cac aat gct atc ctg gac ttt gag cac cgc aca       336
   Asp Val Leu Gln Asp His Asn Ala Ile Leu Asp Phe Glu His Arg Thr
               100                 105                 110
   tcg acc ctg aaa ggg aag aag ttt cgc ctt ctg cct gtg gga ggg tcc       384
   Ser Thr Leu Lys Gly Lys Lys Phe Arg Leu Leu Pro Val Gly Gly Ser
           115                 120                 125
   ctg gaa gat gag ttt gac ctg gag                                       408
   Leu Glu Asp Glu Phe Asp Leu Glu
       130                 135
<210> 25
   <211> 172
   <212> PRT
   <213> Homo Sapiens
<400> 25
   Leu Leu Lys Ala Phe Gly Val Pro Gly Ala Ala Pro Ser His Leu Pro
   1               5                   10                  15
   Lys Glu Ile Val Met Gly Lys Gly Tyr Tyr Leu Lys Gly Lys Ile Gly
               20                  25                  30
   Lys Val Pro Val Arg Phe Leu Val Asp Ser Gly Ala Gln Val Ser Val
           35                  40                  45
   Val His Pro Asn Leu Trp Glu Glu Val Thr Asp Gly Asp Leu Asp Thr
       50                  55                  60
   Leu Gln Pro Phe Glu Asn Val Val Lys Val Ala Asn Gly Ala Glu Met
   65                  70                  75                  80
   Lys Ile Leu Gly Val Trp Asp Thr Ala Val Ser Leu Gly Lys Leu Lys
                   85                  90                  95
   Leu Lys Ala Gln Phe Leu Val Ala Asn Ala Ser Ala Glu Glu Ala Ile
               100                 105                 110
   Ile Gly Thr Asp Val Leu Gln Asp His Asn Ala Ile Leu Asp Phe Glu
           115                 120                 125
   His Arg Thr Cys Thr Leu Lys Gly Lys Lys Phe Arg Leu Leu Pro Val
       130                 135                 140
   Gly Gly Ser Leu Glu Asp Glu Phe Asp Leu Glu Leu Ile Glu Glu Asp
   145                 150                 155                 160
   Pro Ser Ser Glu Glu Gly Arg Gln Glu Leu Ser His
                   165                 170
<210> 26
   <211> 516
   <212> DNA
   <213> Homo sapiens
<220>
   <221> CDS
   <222> (1) .. (516)
   <223>
<400> 26
   ctc ctg aag gcc ttt ggg gtc cct ggg gct gcc ccc agc cac ctg ccc      48
   Leu Leu Lys Ala Phe Gly Val Pro Gly Ala Ala Pro Ser His Leu Pro
   1               5                   10                  15
   aaa gag atc gtc atg ggt aag ggc tac tat ctc aag ggg aag att ggc      96
   Lys Glu Ile Val Met Gly Lys Gly Tyr Tyr Leu Lys Gly Lys Ile Gly
               20                      25                      30
   aaa gtg ccc gtg agg ttc ctg gtg gac tct ggg gcc cag gtc tct gtg         144
   Lys Val Pro Val Arg Phe Leu Val Asp Ser Gly Ala Gln Val Ser Val
           35                  40                  45
   gtc cac cca aac ttg tgg gag gag gtc act gat ggc gat ctg gac acc         192
   Val His Pro Asn Leu Trp Glu Glu Val Thr Asp Gly Asp Leu Asp Thr
       50                  55                  60
   ctg cag ccc ttt gag aat gtg gta aag gtg gcc aat ggt gct gaa atg         240
   Leu Gln Pro Phe Glu Asn Val Val Lys Val Ala Asn Gly Ala Glu Met
   65                  70                  75                  80
   aag atc ctg ggt gtc tgg gat aca gcg gtg tcc cta ggc aag ctg aag         288
   Lys Ile Leu Gly Val Trp Asp Thr Ala Val Ser Leu Gly Lys Leu Lys
                   85                  90                  95
   ctg aag gca cag ttc cta gtg gcc aat gcg agt gcc gag gaa gcc atc         336
   Leu Lys Ala Gln Phe Leu Val Ala Asn Ala Ser Ala Glu Glu Ala Ile
               100                 105                 110
   att ggc act gat gtg ctc cag gac cac aat gct atc ctg gac ttt gag         384
   Ile Gly Thr Asp Val Leu Gln Asp His Asn Ala Ile Leu Asp Phe Glu
           115                 120                 125
   cac cgc aca tgc acc ctg aaa ggg aag aag ttt cgc ctt ctg cct gtg         432
   His Arg Thr Cys Thr Leu Lys Gly Lys Lys Phe Arg Leu Leu Pro Val
       130                 135                 140
   gga ggg tcc ctg gaa gat gag ttt gac ctg gag ctc ata gag gag gac         480
   Gly Gly Ser Leu Glu Asp Glu Phe Asp Leu Glu Leu Ile Glu Glu Asp
   145                 150                 155                 160
   ccc tcc tca gaa gaa ggg cgg cag gag cta tcc cac                         516
   Pro Ser Ser Glu Glu Gly Arg Gln Glu Leu Ser His
                   165                 170
<210> 27
   <211> 155
   <212> PRT
   <213> Homo Sapiens
<400> 27
   Leu Leu Lys Ala Phe Gly Val Pro Gly Ala Ala Pro Ser His Leu Pro
   1               5                   10                  15
   Lys Glu Ile Val Met Gly Lys Gly Tyr Tyr Leu Lys Gly Lys Ile Gly
               20                  25                  30
   Lys Val Pro Val Arg Phe Leu Val Asp Ser Gly Ala Gln Val Ser Val
           35                  40                  45
   Val His Pro Asn Leu Trp Glu Glu Val Thr Asp Gly Asp Leu Asp Thr
       50                  55                  60
   Leu Gln Pro Phe Glu Asn Val Val Lys Val Ala Asn Gly Ala Glu Met
   65                  70                  75                  80
   Lys Ile Leu Gly Val Trp Asp Thr Ala Val Ser Leu Gly Lys Leu Lys
                   85                  90                  95
   Leu Lys Ala Gln Phe Leu Val Ala Asn Ala Ser Ala Glu Glu Ala Ile
               100                 105                 110
   Ile Gly Thr Asp Val Leu Gln Asp His Asn Ala Ile Leu Asp Phe Glu
           115                 120                 125
   His Arg Thr Cys Thr Leu Lys Gly Lys Lys Phe Arg Leu Leu Pro Val
       130                 135                 140
   Gly Gly Ser Leu Glu Asp Glu Phe Asp Leu Glu
   145                 150                 155
<210> 28
   <211> 465
   <212> DNA
   <213> Homo sapiens
<220>
   <221> CDS
   <222> (1) .. (465)
   <223>
<400> 28
   ctc ctg aag gcc ttt ggg gtc cct ggg gct gcc ccc agc cac ctg ccc        48
   Leu Leu Lys Ala Phe Gly Val Pro Gly Ala Ala Pro Ser His Leu Pro
   1               5                   10                  15
   aaa gag atc gtc atg ggt aag ggc tac tat ctc aag ggg aag att ggc        96
   Lys Glu Ile Val Met Gly Lys Gly Tyr Tyr Leu Lys Gly Lys Ile Gly
               20                  25                  30
   aaa gtg ccc gtg agg ttc ctg gtg gac tct ggg gcc cag gtc tct gtg       144
   Lys Val Pro Val Arg Phe Leu Val Asp Ser Gly Ala Gln Val Ser Val
           35                  40                  45
   gtc cac cca aac ttg tgg gag gag gtc act gat ggc gat ctg gac acc       192
   Val His Pro Asn Leu Trp Glu Glu Val Thr Asp Gly Asp Leu Asp Thr
       50                  55                  60
   ctg cag ccc ttt gag aat gtg gta aag gtg gcc aat ggt gct gaa atg       240
   Leu Gln Pro Phe Glu Asn Val Val Lys Val Ala Asn Gly Ala Glu Met
   65                  70                  75                  80
   aag atc ctg ggt gtc tgg gat aca gcg gtg tcc cta ggc aag ctg aag       288
   Lys Ile Leu Gly Val Trp Asp Thr Ala Val Ser Leu Gly Lys Leu Lys
                   85                  90                  95
   ctg aag gca cag ttc cta gtg gcc aat gcg agt gcc gag gaa gcc atc       336
   Leu Lys Ala Gln Phe Leu Val Ala Asn Ala Ser Ala Glu Glu Ala Ile
               100                 105                 110
   att ggc act gat gtg ctc cag gac cac aat gct atc ctg gac ttt gag       384
   Ile Gly Thr Asp Val Leu Gln Asp His Asn Ala Ile Leu Asp Phe Glu
           115                 120                 125
   cac cgc aca tgc acc ctg aaa ggg aag aag ttt cgc ctt ctg cct gtg       432
   His Arg Thr Cys Thr Leu Lys Gly Lys Lys Phe Arg Leu Leu Pro Val
       130                 135                 140
   gga ggg tcc ctg gaa gat gag ttt gac ctg gag 465
   Gly Gly Ser Leu Glu Asp Glu Phe Asp Leu Glu
   145 150 155
<210> 29
   <211> 4
   <212> PRT
   <213> Homo sapiens
<400> 29
   Phe Ala Asn Ser
   1
<210> 30
   <211> 6
   <212> PRT
   <213> Homo sapiens
<400> 30
   Asp Leu Glu Leu Ile Glu
   1 5
<210> 31
   <211> 10
   <212> PRT
   <213> Homo sapiens
<400> 31
   Glu Phe Asp Leu Glu Leu Ile Glu Glu Asp
   1 5 10
<210> 32
   <211> 11
   <212> PRT
   <213> Homo sapiens
<400> 32
<210> 33
   <211> 10
   <212> PRT
   <213> Homo sapiens
<400> 33
   Glu Phe Asp Leu Asp Leu Ile Glu Trp Asp
   1 5 10
<210> 34
   <211> 10
   <212> PRT
   <213> Homo sapiens
<400> 34
   Glu Phe Asp Leu Asp Leu Ile His Trp Asp
   1 5 10
<210> 35
   <211> 10
   <212> PRT
   <213> Homo sapiens
<400> 35
   Glu Pro Asn Leu Asp Leu Ile Glu Glu Asp
   1 5 10
<210> 36
   <211> 255
   <212> PRT
   <213> Homo sapiens
<400> 36
   Met Ala Gly Ser Gly Ala Arg Ser Glu Glu Gly Arg Arg Gln His Ala
   1 5 10 15
   Phe Val Pro Glu Pro Phe Asp Gly Ala Asn Val Val Pro Asn Leu Trp
   20 25 30
   Leu His Ser Phe Glu Val Ile Asn Asp Leu Asn His Trp Asp His Ile
   35 40 45
   Thr Lys Leu Arg Phe Leu Lys Glu Ser Leu Arg Gly Glu Ala Leu Gly
   50 55 60
   Val Tyr Asn Arg Leu Ser Pro Gln Asp Gln Gly Asp Tyr Gly Thr Val
   65 70 75 80
   Lys Glu Ala Leu Leu Lys Ala Phe Gly Val Pro Gly Ala Ala Pro Ser
   85 90 95
   His Leu Pro Lys Glu Ile Val Met Gly Lys Gly Tyr Tyr Leu Lys Gly
   100 105 110
   Lys Ile Gly Lys Val Pro Val Arg Phe Leu Val Asp Ser Gly Ala Gln
   115 120 125
   Val Ser Val Val His Pro Asn Leu Trp Glu Glu Val Thr Asp Gly Asp
   130 135 140
   Leu Asp Thr Leu Gln Pro Phe Glu Asn Val Val Lys Val Ala Asn Gly
   145 150 155 160
   Ala Glu Met Lys Ile Leu Gly Val Trp Asp Thr Ala Val Ser Leu Gly
   165 170 175
   Lys Leu Lys Leu Lys Ala Gln Phe Leu Val Ala Asn Ala Ser Ala Glu
   180 185 190
   Glu Ala Ile Ile Gly Thr Asp Val Leu Gln Asp His Asn Ala Ile Leu
   195 200 205
   Asp Phe Glu His Arg Thr Cys Thr Leu Lys Gly Lys Lys Phe Arg Leu
   210 215 220
   Leu Pro Val Gly Gly Ser Leu Glu Asp Glu Phe Asp Leu Glu Leu Ile
   225 230 235 240
   Glu Glu Asp Pro Ser Ser Glu Glu Gly Arg Gln Glu Leu Ser His
   245 250 255